# EUROPEAN PATENT APPLICATION

(11) **EP 4 613 280 A1**
(43) Date of publication of application: **10.09.2025**
(21) Application number: 24305330.3
(22) Date of filing: 04.03.2024
(51) Int. Cl.: A61K 36/21, A61K 36/28, A61K 36/31, A61K 39/395, A61P 35/00, C07K 16/00, C12N 5/00, A61K 9/00

(54) **PLANT MATERIAL FOR USE AS MEDICAMENT**

(71) Applicant: Plantibodies, 75005 Paris (FR)
(72) Inventor: MAZHAR, Bilal, 75013 PARIS (FR); BAUËR, Pierre, 75005 PARIS (FR)
(74) Representative: Plasseraud IP

(57) **Abstract**

The present invention relates to a plant material for use as medicament, wherein the plant material is derived from a plant of the *Brassica, Spinacia,* or *Lactuca* genus, genetically modified to express a biologically active recombinant protein and comprises the biologically active recombinant protein as an active ingredient. The invention also relates to a method for producing such a plant material, and to a pharmaceutical composition comprising such a plant material and an orally acceptable support.

## Description

### Background

To treat chronic diseases, the biopharma industry strives to develop new and innovative classes of drugs. Historically they have developed chemotherapy drugs to treat and retard cancerous growth. However, in recent decades new strategies such as immunotherapies using monoclonal antibodies have been developed. These antibodies are typically produced using mammalian cell lines (HeLa, CHO, etc.). Yet, this approach has limitations, due to complex infrastructural needs and operation costs.

The production of therapeutic proteins by plants is an area that has been explored for its potential to reduce costs. However, the production of proteins in plants poses a whole series of problems. The biological activity and yield constitute a first series issues in the field of therapeutic proteins produced in plants, especially in the field in the case of complex multimeric proteins such as antibodies. The model plants that have been worked on the most in response to these problems are tobacco, tomato and arabidopsis, with the result that today most of the plant-produced recombinant proteins and in particular recombinant antibodies have been obtained from genetically modified tobacco plants or cell lines. Furthermore, to optimize yields, competitors have often favored transient expression.

In that respect, the applicant is at the origin of the development of an alternative heterologous expression system based on genetically modified plant selected from the *Spinacia, Lactuca,* and *Brassica* genus, that provides for the stable expression of a protein of interest into the plant. Application WO 2023/036984 A1 relates to a method for producing a genetically modified plant selected from the *Spinacia, Lactuca,* and *Brassica* genus, comprising a step of introducing a plant nucleic acid construct that provides for stable expression of the protein of interest, including that of multimeric proteins such as antibodies, wherein the nucleic acid is introduced in the form of a single synthetic construct comprising a regulatory sequence active in a plant for the expression of an operably linked DNA, an operably linked protein-coding DNA molecule, and a 3' untranslated region.

The immunogenicity of therapeutic proteins produced in plants is also an important consideration in the development and use of plant-based biopharmaceuticals. Immunogenicity refers to the ability of a substance to provoke an immune response in the body. In the context of therapeutic proteins produced in plants, several factors that can influence their immunogenicity are the differences between plant and mammals in glycosylation patterns and post-translational modifications, and the risk of protein aggregation of the plant-based therapeutic protein. To address these concerns and minimize immunogenicity of plant-based therapeutic proteins, numerous strategies have been developed such as glycoengineering (modifying the plant glycosylation patterns to resemble more closely human glycosylation) and purification techniques to help remove fractions that may cause immunogenicity including protein aggregates and plant-specific impurities such as endotoxins.

A common limit to the techniques set out above to reduce immunogenicity of therapeutic proteins produced in plants lies within their difficulty of implementation and the risk of resulting in lower yields or in lower biological activity of the plant produced recombinant protein.

The administration of therapeutic proteins to the intestine is explored for various diseases and medical conditions. It is a challenging task due to the harsh conditions of the gastrointestinal tract, including the acidic environment of the stomach and the presence of proteolytic enzymes. Various strategies have been developed to overcome these challenges and deliver therapeutic proteins to the intestine. These include enteric coating, protein engineering, and/or encapsulation in nanoparticles or microspheres. Enteric coatings are designed to protect therapeutic proteins from the acidic environment of the stomach and to allow them to reach the intestine intact, where absorption can occur. Engineering of proteins involves modifications to the protein structure, such as glycosylation or PEGylation, can improve stability and protect against degradation. Encapsulation of therapeutic proteins in nanoparticles or microspheres, provide protection during transit through the stomach, and the particles can be designed to release the protein in a controlled manner in the intestine, enhancing absorption. While the techniques set out above allow for the delivery of therapeutic proteins, there are also important drawbacks associated with these approaches: enteric coating and more generally encapsulation involve specific materials and complex manufacturing processes, resulting in high production costs and frequently in scaling up challenges. Moreover, proteins engineering often pose challenges in terms of immunogenicity. The size of proteins that can be delivered is also a limiting factor. in particular, the larger the proteins, the more difficult it is to stably encapsulate them. The delivery of complex proteins like antibodies to the intestine is particularly challenging.

Therefore there is still a need to overcome the drawbacks of the prior art and to provide new strategies to deliver a biologically active protein, in particular a complex multimeric protein such as an antibody, in active form to the intestine, without causing immunogenicity, that can be implemented at large-scale with low-cost production.

### Brief description

The present invention is at least partly based on the inventors surprising finding that in the context of model of colorectal cancer model in mice, a lyophilized cabbage plant material genetically modified to express a monoclonal plant-derived antibody, nivolumab, could be used as an oral medicament for treating colorectal cancer.

Thus, in a first object, the disclosure relates to a plant material for use as medicament, wherein the plant material is derived from a plant of the *Brassica, Spinacia,* or *Lactuca* genus, genetically modified to express a biologically active recombinant protein and comprises the biologically active recombinant protein as an active ingredient.

In some embodiments, the plant material is from an edible plant from the *Brassica* genus, preferably is from cabbage.

In some embodiments, the plant material is a dried plant material, preferably is a homogenate of dried plant material.

In some embodiments, the biologically active recombinant protein is a multimeric protein.

In some embodiments, the biologically active recombinant protein is an antibody or a fragment thereof.

In some embodiments, the plant material is administered orally.

In some embodiments, the plant material is for use in a method for treatment of an intestinal disorder in a subject.

In some embodiments, the plant material delivers the biologically active recombinant protein, under active form, to the lumen or mucosal surface of the intestine of the subject.

In some embodiments, the plant material is for use in a method for treatment of an intestinal disorder in a subject, wherein the intestinal disorder is selected from cancer, infectious diseases and auto-immune diseases.

In some embodiments, the plant material is for use in a method for treatment of an intestinal disorder in a subject, wherein the intestinal disorder is colorectal cancer.

In some embodiments, the biologically active recombinant protein is an antibody selected from an anti-PD1 antibody, an anti-PDL1 antibody, an anti-CTLA4 antibody or a fragment thereof.

Typically, the biologically active recombinant protein is selected from nivolumab and pembrolizumab.

In a second object, the disclosure relates to a method for producing a plant material according to the first object, the method comprising:
a) providing a plant of the *Brassica, Spinacia,* or *Lactuca* genus stably transformed to express the biologically active recombinant protein;
b) harvesting a plant material from the plant of step a).

In some embodiments, the method further comprises the steps of:
c) drying the plant material, preferably by freeze-drying or lyophilization,
d) homogenizing the plant material.

In a third aspect, the disclosure relates to a pharmaceutical composition comprising the plant material according to the first aspect, and an orally acceptable support.

### Detailed description

### 1. Plant material for use as medicament

The inventors have shown that surprisingly, in the context of a colorectal cancer model in mice, a lyophilized homogenate of cabbage plant material genetically modified to express a monoclonal antibody, nivolumab, could be used as an oral medicament for treating colorectal cancer. Indeed, the inventors have shown that when administered orally, said plant material was able to deliver the nivolumab antibody to intestinal tumors in quantities sufficient to reduce colorectal tumor growth.

This means that, surprisingly, the plant material not only provides a protective effect potent enough to protect the nivolumab antibody from the harsh conditions in the gastrointestinal tract, but also, and even more surprisingly, that is also able to deliver the antibody in active form to tumor sites in the lumen of the intestine.

Without wanting to be bound by any theory, the inventors believe that the protective effect is due to the fact that when administered orally, the plant material comprises the monoclonal antibody encapsulated in the plant cells, and that quite unexpectedly, this encapsulation turns to be sufficient to provide enough protection to fragile and complex multimeric proteins such as antibodies against the harsh conditions of the gastrointestinal tract.

They also have demonstrated that administration of said plant material was well tolerated by the mice since it did not impact main metabolic parameters associated to liver, kidneys, and the pancreas (e.g. creatinine, ureum, tryglicerides) nor the glucose level in the blood.

Remarkably, the inventors have shown that the treated mice demonstrated increased levels of fecal IgM and serum IgA, suggesting a humoral immune response by intestinal immune cells. Without wanting to be bound by any theory, the inventors believe that the IgM antibodies can be a response from intestinal B lymphocytes, while serum IgA reflects on activity by the intestinal plasma cells, and that a B cell response could have originated from the lamina propria or cells resident in the intestinal lymph nodes.

Thus, in a first object, the disclosure relates to a plant material for use as medicament, wherein the plant material is derived from a plant of the *Brassica, Spinacia,* or *Lactuca* genus genetically modified to express a biologically active recombinant protein and comprises the biologically active recombinant protein as an active ingredient.

### Plant material

The plant material is derived from a plant from the *Brassica, Spinacia,* or *Lactuca* genus genetically modified to express a biologically active protein.

The term "'plant" as used herein encompasses whole plants, ancestors and progeny of the plants and plant parts, including seeds, leaves, shoots, stems, roots (including tubers), plant cells, protoplasts, tissues and organs. The plant may be in any form including suspension cultures, embryos, meristematic regions, callus tissue, leaves, gametophytes, sporophytes, pollen, and microspores. The plant is preferably a whole plant.

The term "plant material derived from a plant" or "plant material" as used herein refers to any part or tissue of a plant that can be harvested from a plant, i.e. fresh plant materials including leaves, stems, roots, seeds, fruits and flowers and plant materials derived from such plant materials, such as homogenate of plant materials, dried plant materials, or dried homogenates of plant materials. Such plant materials comprise plant cells.

The term "plant cells" as used herein refers to the basic structural and functional units of plants. Plant cells have several distinctive structures including cell walls, vacuoles, chloroplasts, Endoplasmic Reticulum (ER), Golgi apparatus, and nucleus.

In some embodiments, the plant material comprises the biologically active recombinant protein encapsulated in plant cells.

As used herein, "encapsulated in plant cells", referring to the biologically active recombinant proteins, means that the biologically recombinant protein is enclosed or contained within the cells of the plant material. This encapsulation might involve the trapping or surrounding of the biologically active recombinant protein within the cellular structures of plant cells. Without wanting to be bound by any theory, the inventors believe that the biologically active recombinant protein is in the endoplasmic reticulum, the Golgi, and/or the cytoplasm of the plant cells of the plant material.

In some embodiments, the plant material is a crude plant material, for example is crude leaves.

In some embodiments, the plant material is a homogenate of plant material, for example is a homogenate of leaves.

As used herein, the term "homogenate" refers to a mixture or suspension of plant material tissue components that have been thoroughly disrupted or broken down to create a uniform and consistent blend. As detailed below, the process of homogenization may involves mechanically or enzymatically breaking down the plant material samples to produce a homogenous mixture.

In some embodiments, the plant material is a homogenate of dried plant material, for example is a homogenate of dried leaves.

The term " homogenate of dried plant material" or "dried homogenate" as used herein refers to a homogenized sample of plant material that has been subjected to a drying process, such as freeze drying or lyophilization. Drying processes are further described below.

Preferably, the plant material is a homogenate of dried plant leaves, more preferably is a homogenate of lyophilized plant leaves.

### Brassica, Spinacia, and Lactuca genus

The genetically modified plant material is from the *Brassica, Spinacia,* or *Lactuca* genus.

The term *"Brassica* genus" as used herein encompasses a group of plants commonly known as the mustard family (Brassicaceae), also known as the cruciferous or cabbage family. This genus includes several important vegetable crops and oilseed crops.

Brassicaceae contains some 338 genera and more than 3,700 species of flowering plants. Brassicaceae species are characterized by four-petalled cross-shaped flowers that feature two long and two short stamens and produce pod like fruits known as siliques. Among the Brassicaceae family, cabbage plants and relative, from the *Brassica* genus, are of particular relevance. *Brassica* are leafy green, red (purple), or white (pale green) plants within the Brassicaceae family. *Brassica* plants are commonly grown in agriculture and horticulture as a food source because of their dense-leaved heads. Most of the *Brassica* genus plants are annual or biennials. Some of the commonly recognized vegetables within this genus are mustards, Canola, Broccoli, Cauliflower, Cabbages, Choy Sum, and Brussels sprouts. Generally grown cabbages weigh within a range of 500 to 1000 grams. They are cultivated in well-drained soils with a pH between 6 and 8, receiving full sun. These growing conditions allow the plants to develop densely leaved heads. The plants require adequate levels of phosphorus, potassium, and nitrogen in the soil, especially during the early stages of growth. These plants grow best between 4 and 24 °C. A temperature variation may cause the plant to blot and produce flowers. In some embodiments, lower temperatures may cause vernalization of the flowers. Hence, *Brassica* can be planted at the beginning of the cold period and survive until a later warm period before inducing flowers.

In some embodiments, the plant material is from an edible plant from the *Brassica* genus, preferably is from cabbage.

Cabbage plants notably include bok choy (*Brassica rapa, variety chinensis*), brown mustard (*Brassica juncea*), broccoli (*Brassica oleracea, variety italica*), Brussels sprouts (*Brassica* oleracea, variety gemmifera), cabbage (*Brassica oleracea, variety capitata*), cauliflower (*Brassica oleracea, variety botrytis*), collard (*Brassica oleracea, variety acephala*), kale (*Brassica* oleracea, variety acephala), kohlrabi (*Brassica* oleracea, variety gongylodes), napa cabbage (*Brassica rapa, variety pekinensis*), rape (*Brassica napus, variety napus*), rutabaga (*Brassica napus, variety napobrassica*), and turnip (*Brassica rapa, variety rapa*).

The term "*Spinacia* genus" as used herein encompasses a group of flowering plants in the family Amaranthaceae. The most well-known species in this genus is *Spinacia oleracea,* commonly known as spinach. Spinach is a leafy green vegetable that is widely cultivated for its nutritious leaves.

The term "*Lactuca*" as used herein encompasses a group of plants in the Asteraceae family, commonly known as the lettuce family. The most well-known and widely cultivated species in this genus is *Lactuca sativa,* which is simply known as lettuce. Lettuce is a popular leafy vegetable that is commonly used in salads and various culinary dishes.

### Genetically modified plant

The plant is genetically modified to express a recombinant biologically active protein.

The expression "genetically modified plant or "transgenic plant" refers to a plant or progeny thereof derived from a transformed plant cell, protoplast, or other transformed plant tissue wherein the plant DNA contains an introduced exogenous DNA molecule not originally present in a corresponding native, non-transgenic plant of the same species.

The expression "the plant is genetically modified", "genetically modified plant or "transgenic plant" as used herein refers to a plant or progeny thereof derived from a transformed plant cell, protoplast, or other transformed plant tissue wherein the plant DNA contains an introduced exogenous DNA molecule not originally present in a corresponding native, non-transgenic plant of the same species.

As used herein, "express a recombinant biologically active protein", "transgene expression", "expressing a transgene", "protein expression", and "expressing a protein" refers to the production of a protein through the process of transcribing a DNA molecule into messenger RNA (mRNA) and translating the mRNA into polypeptide chains, which may or may not be ultimately folded into proteins.

In some embodiments, the plant stably expresses the biologically active protein.

The term "stable expression" as used herein refers to a long-term production of the recombinant protein in the host cell plant or plant. This is in contrast to a transient expression, where the recombinant protein is produced for a limited period.

### Biologically active recombinant protein

The plant material comprises the biologically active protein as an active ingredient.

As used herein, the term "active ingredient" refers to the component of a medication responsible for producing the desired therapeutic effect. It is a pharmacologically active substance that directly interacts with the subject to treat a specific condition, alleviate symptoms, or achieve a desired outcome.

In some embodiments, the biologically active recombinant protein is a multimeric protein, for example an antibody or a fragment thereof.

In some embodiments, the biologically active recombinant protein has a molecular weight comprised between 10 kDa and 170 kDa, between 10 kDa and 150 kDa, between 60 kDa and 170 kDa, or between 60 kDa and 150 kDa.

In some embodiments, the biologically active recombinant protein is a multimeric protein, for example the antibody or fragment thereof, having a molecular weight comprised between 130 and 170 kDa, preferably between 140 and 160 kDa, more preferably between 145 and 150 kDa.

In some embodiments, the plant material comprises between 0.01 and 10 mg, 0.1 and 10 mg, 2 and 10 mg of biologically active recombinant protein, preferably between 2 and 10 mg of biologically active recombinant protein, preferably between 2 and 6 mg of biologically active recombinant protein, per g of fresh weight of plant material, wherein the biologically active recombinant protein is preferably a multimeric protein, for example the antibody or fragment thereof. This corresponds to a plant material comprising between 0.001% and 1%, preferably between 0.01% and 1%, preferably between 0.2% and 1%, preferably between 0.2% and 0.6% of biologically active recombinant protein, % expressed in weight relative to the fresh weight of plant material.

When the plant material undergoes a drying process, the extraction of water, either in part or entirely, results in a notable augmentation in biologically active recombinant protein concentration, for example increasing by a factor of at least 10-fold, typically by a factor ranging between 10-fold and 20-fold.

In some embodiments, the plant material comprises between 0.05 and 100 mg of biologically active recombinant protein, preferably between 0.05 and 50 mg of biologically active recombinant protein 0.05 and 25 mg of biologically active recombinant protein, 0.05 and 50 mg of biologically active recombinant protein, per g of dry weight of plant material, wherein the biologically active recombinant protein is preferably a multimeric protein, for example the antibody or fragment thereof. This corresponds to a plant material comprising between 0.005% and 10%, preferably between 0.005% and 5%, preferably between 0.005% and 2.5% of biologically active recombinant protein, % expressed in weight relative to the dry weight of plant material.

In some embodiments of the present disclosure, the biologically active recombinant protein, for example the antibody or fragment thereof, is for use in in cancer therapy, against infectious diseases or in auto-immune disease's therapy.

In some embodiments, the antibody can be directed against bacterial, viral, fungal or cancer antigens. Cancer antigens are antigens expressed in the context of a cancer that can be targeted for cancer therapy. In some embodiments, the antibody is directed against a tumor antigen, or an immune checkpoint molecule (in particular an inhibitory immune checkpoint molecule). Examples of relevant immune checkpoint molecules include PD1, PDL1, CTLA4, EGFR, LAG3, BTLA, OX2R, TIM-3, TIGIT, LAIR-1, PGE2 receptor, EP2/4 adenosine receptor, or A2AR.

In some embodiments, the antibody is an anti-PD1 antibody, an anti-PDL1 antibody, an anti-EGFR antibody, an anti-CTLA4 antibody, or a fragment thereof.

Anti-PD-1 antibodies are a class of immunotherapy drugs designed to treat various cancers. PD-1, or programmed cell death protein 1, is a cell surface receptor found on certain immune cells, including T cells. When PD-1 binds to its ligands, PD-L1 (programmed death-ligand 1) or PD-L2, it inhibits the activity of T cells, preventing them from attacking normal cells, including cancer cells. Cancer cells can exploit this mechanism to evade the immune system. By blocking the interaction between PD-L1 and its receptor PD-1 (Programmed Cell Death Protein 1) on immune cells, anti-PD1 and anti-PD-L1 antibodies aim to enhance the immune system's ability to recognize and attack cancer cells.

Examples of anti-PD1 antibodies include Nivolumab (Opdivo), Pembrolizumab (Keytruda), and Cemiplimab (Libtayo).

Examples of anti-PDL1 antibodies include Atezolizumab (Tecentriq), Durvalumab (Imfinzi) and Avelumab (Bavencio).

Anti-EGFR (Epidermal Growth Factor Receptor) antibodies are a class of therapeutic antibodies that target the EGFR, a cell surface receptor that plays a crucial role in cell growth and survival. EGFR is overexpressed or mutated in various cancers, including colorectal cancer, non-small cell lung cancer, and head and neck cancers. Blocking EGFR with antibodies can inhibit the growth of cancer cells.

Examples of anti-EGFR antibodies include Cetuximab (Erbitux) and Panitumumab (Vectibix).

Anti-CTLA-4 antibodies refer to antibodies that target cytotoxic T-lymphocyte-associated protein 4 (CTLA-4), a protein that plays a crucial role in regulating the immune system. CTLA-4 is expressed on the surface of T cells, and its main function is to downregulate immune responses by inhibiting T cell activation. By blocking CTLA-4, anti-CTLA-4 antibodies enhance the activity of T cells, particularly cytotoxic T cells, allowing for a more robust immune response against cancer cells.

Examples of anti-CLTA-4 antibodies include Ipilimumab (Yervoy).

In some embodiments, the antibody is selected from the group constituted of Nivolumab, Pembrolizumab, Cemiplimab, Atezolizumab, Durvalumab, Avelumab, Cetuximab, Panitumumab, Ipilimumab, and fragments thereof.

In some embodiments, the antibody is selected from the group constituted of Nivolumab, Pembrolizumab, Cemiplimab, Atezolizumab, Durvalumab, Avelumab, and fragments thereof.

In some embodiments, the antibody is selected from the group constituted of Nivolumab, Pembrolizumab, and fragments thereof.

### Method of treatment

As used herein, the term "for use as a medicament" means that the plant is for use in a method for treatment of the human or animal body by therapy.

As used in this document, the term "treatment" or "therapy" refers to any action which makes it possible to reduce or suppress the symptoms associated with a pathological condition. It comprises both a curative treatment and a prophylactic treatment for a disease.

A curative treatment is defined by a treatment resulting in a cure or a treatment which relieves, improves and/or eliminates, reduces and/or stabilizes the symptoms of a disease or the suffering that it causes. The term "curative treatment" may refer to one or more of (1) inhibiting the disease; for example, inhibiting a disease, condition or disorder in an individual who is experiencing or displaying the pathology or symptomatology of the disease, condition or disorder (i.e., arresting further development of the pathology and/or symptomatology); and (2) ameliorating the disease; for example, ameliorating a disease, condition or disorder in an individual who is experiencing or displaying the pathology or symptomatology of the disease, condition or disorder (i.e., reversing the pathology and/or symptomatology) such as decreasing the severity of disease or reducing or alleviating one or more symptoms of the disease. A prophylactic treatment comprises both a treatment resulting in the prevention of a disease and a treatment which reduces and/or delays the incidence of a disease or the risk of it occurring. The terms "improve" and "reduce" include, but do not require complete recovery or complete prevention. The term "prophylactic treatment" may refer to one or more of (1) preventing the disease; for example, preventing a disease, condition or disorder in an individual who is at risk of experiencing or displaying the pathology or symptomatology of the disease, condition or disorder (i.e., preventing the development of the pathology and/or symptomatology); and (2) reducing and/or delaying the incidence of a disease in an individual who is experiencing or displaying the pathology or symptomatology of the disease, condition or disorder (i.e., reversing the pathology and/or symptomatology) such as decreasing the severity of disease or reducing or alleviating one or more symptoms of the disease. In particular, a prophylactic treatment may refer to a treatment administered to a subject to prevent a relapse after a period of initial treatment and apparent remission.

In the context of the present disclosure, the term "treatment" preferably refers to a curative or prophylactic treatment which is administered orally and/or to a curative or prophylactic treatment of an intestinal disorder, as further described herein.

The disclosure also provides the use of a plant material as described herein optionally in association with an orally acceptable support and/or one or more active substance as described herein for the manufacture of a medicament, in particular of a medicament for oral administration, in particular of a medicament use in treatment of an intestinal disorder as described herein.

The disclosure also provides a method of treatment, in particular by oral administration and/or for the treatment of an intestinal disorder, the method comprising administering to a subject in need thereof an effective amount of plant material as describe herein optionally in association with a pharmaceutically acceptable support and/or one or more active substance as described herein.

### Route of Administration

The plant material according to the disclosure is preferably for oral administration.

As used herein, oral administration refers to the route of drug delivery in which medications are taken through the mouth and swallowed. This route of administration is particularly advantageous since it is a convenient way to administer a drug which does not require hospitalization (unlike the intravenous route) and because it is non-invasive.

In other word, the plant material is preferably for use as an oral medicament.

The disclosure thus provides an orally acceptable and well-tolerated plant material as a delivery system for delivering a biologically active recombinant protein.

As used herein, the term "delivery system" or "protein delivery system" refers to a method or technology designed to efficiently transport proteins to specific target sites within the body, such as cells, tissues, or organs. In the present disclosure, the targeted organ is the intestine, more especially the lumen or mucosal surface of the intestine.

### Intestinal delivery

The biologically active recombinant protein can be delivered to the intestines, preferably to the anaerobic sections of the intestines, preferably the large intestines and preferably the colon.

The biologically active recombinant protein can be delivered such that it is not damaged despite the harsh conditions of the GI tract, in particular the acidic environment of the stomach and the presence of proteolytic enzymes.

The term "GI tract" as used herein is a long tube that extends from the mouth to the anus. It comprises digestive structures including the esophagus, stomach, and intestines. The GI tract does not include the accessory glandular organs such as the liver, biliary tract or pancreas. The intestines include the small intestine and large intestine. The small intestine includes the duodenum, jejunum and ileum. The large intestine includes the cecum, colon, rectum and anus. The upper GI tract includes the buccal cavity, pharynx, esophagus, stomach, and duodenum. The lower GI tract includes the small intestine below the duodenum and the large intestine. Preferably, the plant material of the disclosure delivers the biological active recombinant protein to the lumen or mucosal surface of the intestine, more preferably the lumen or mucosal surface of the large intestine, and more preferably the lumen or mucosal surface of the colon.

In some embodiments, the plant material delivers the biologically active recombinant protein, under active form, to the lumen or mucosal surface of the intestine of the subject, preferably at least to the lumen or mucosal surface colon of the subject.

In some embodiments, the plant material delivers the biologically active recombinant protein, under active form, to at least part of the lumen or mucosal surface of the small intestine, in some embodiments at least to the lumen or mucosal surface of the end part of the small intestine of the subject.

In some embodiments, the biologically active recombinant protein is not delivered to the esophagus and/or stomach.

The disclosure also provides pharmaceutical compositions comprising the plant material that can be administered orally, as detailed further in the third object of the disclosure.

In some embodiments, the biologically active recombinant protein is delivered to the site in the GI tract associated with cancer, infectious disease or auto-immune disease, where it may exhibits a local and/or a systemic effect.

As used herein, the term "local effect" means that the effect is confined to the immediate area of drug administration, absorption, or delivery and typically does not involve systemic distribution throughout the body of the subject.

As used herein, the term "systemic effect" involves that the effect is not limited to the area of drug administration, absorption, or delivery and reaches various tissues and organs.

In some embodiments, the biologically active recombinant protein is delivered to the site in the GI tract associated with cancer, infectious disease or auto-immune disease, where it exhibits a local effect and little or no systemic effect.

Without wanting to be bound by any theory, the inventors believe that that delivering the biologically active recombinant protein locally within the GI tract may allow for dose reduction compared to what would be needed with systemic administration, typically via intravenous injection. This is particularly advantageous, since it mitigates or avoids the adverse side effects often linked to the systemic delivery and uptake of therapeutically effective amounts of antibodies, such as hepatic toxicity.

In that respect, the inventors have shown that the administration of a plant material according to the present disclosure containing nivolumab as biologically active recombinant protein does not cause adverse side effects, in particular does not cause hepatic toxicity or immunogenicity.

### Intestinal disorder therapy

The plant material according to the disclosure is preferably for use in a method for the treatment of an intestinal disorder in a subject.

As used in the present disclosure, the term "intestinal disorder" or "intestine disorder" relates to a disorder affecting the intestines of a subject. It encompasses a wide range of conditions that can affect the structure and function of the intestines, including cancer, infectious diseases and auto-immune diseases.

These disorders can involve the small intestine, large intestine (colon), or both.

In certain embodiments, the intestinal disorder involves the colon.

In some preferred embodiments, the present disclosure relates to the treatment of an intestinal cancer in a subject.

The term "intestinal cancer" as used herein, typically refers to cancer that originates in the intestines, which are part of the digestive system. There are two main types of intestinal cancer: colon cancer and rectal cancer. Collectively, they are often referred to as colorectal cancer.

As used herein, "colon cancer" refers to a type of cancer that develops in the colon, which is the longest part of the intestine. Colon cancer often starts as small, noncancerous clumps of cells called polyps. Over time, some of these polyps can become cancerous, leading to the formation of malignant tumors.

As used herein, "rectal cancer" refers to a type of cancer that develops in the rectum. Like colon cancer, rectal cancer can also develop from precancerous polyps.

In some embodiments, the intestinal cancer is colorectal cancer.

In some embodiments, the intestinal cancer is colon cancer.

In some embodiments, the intestinal cancer is rectal cancer.

With reference to the treatment of a cancer, the term "treatment" may refer to the inhibition of the growth of the tumor, the reduction of the size of the tumor, or the total destruction of the tumor. It can also relate to the prevention of onset or recurrence of cancer.

As used herein, the term "onset of cancer" refers to the initial occurrence of cancer disease within an individual's body, typically the moment when cancerous cells begin to proliferate and grow uncontrollably, forming tumors or affecting normal bodily functions.

As used herein, the term "cancer recurrence" or "cancer relapse" refers to the recurrence of cancer after a period of apparent remission following initial treatment, for example a subject that has undergone successful treatment, including surgery to remove the primary tumor, chemotherapy, or other therapies. The likelihood of relapse can depend on various factors, including the stage of the cancer at the time of initial diagnosis, the success of the treatment, and individual characteristics. Despite successful treatment and the absence of visible tumors, there may be microscopic residual disease that is undetectable. Over time, these residual cells can multiply and lead to relapse.

With reference to cancer relapse, the terms "treatment" or "prevention" may refer to the inhibition of the appearance of a tumor or in other terms to prevent tumors from forming.

In certain embodiments, the disclosure relates to a plant material for use in a method for treating an intestinal disorder in a subject, wherein the plant material is for use in a method for preventing the relapse of an intestinal cancer, preferably of a colon cancer relapse, in a subject.

### Dose

The plant material according to the disclosure is preferably administered to the patient at an effective dose. The term "effective dose" or "therapeutically effective dose" as used herein refers to the amount required to observe the desired curative or prophylactic activity.

In some embodiments, "effective dose" may refer to an amount required to induce an immune response.

In the context of cancer, "effective dose" may refer to an amount required to activate the subject immune system against the tumor cells, i.e. involves activating the subject immune system to recognize and/or attack cancer cells. In some embodiments, it may refer to an amount required to observe an inhibition or a reduction of tumor growth and/or to the prevention of cancer relapse. The amount of plant material to be administered and the duration of the treatment are evaluated by those skilled in the art according to criteria such as the physiological condition of the subject to be treated, the nature of the intestinal disorder to be treated.

The plant material according to the disclosure can be administered in the form of a single dose or multiple doses.

### Subject selection

The subject to be treated, or patient, is an animal, preferably a mammal.

According to some preferred embodiments, the subject to be treated is a human.

According to some embodiments, the subject to be treated is an animal other than human, preferably a domestic animal selected from the group consisting of a bird, in particular a poultry, a fish, a dog, a cat, a horse, a cow, a sheep, a pig and a non-human primate.

### Administration regimen

In some embodiments, the plant material as described herein is or are administered as a single dose, or in a fractionated dose regimen, simultaneously, separately, or sequentially.

In some embodiments, the plant material as described herein is administered to the subject in a fractionated dose regimen.

In some embodiments, the fractionated dose regimen as described herein comprises 2 to 10 fractionated doses.

In a preferred embodiment, the fractionated dose regimen as described herein is administered once daily or once every two days.

### 2. Method for producing a plant material

In a second object, the present disclosure relates to a method for producing a plant material as defined in the first object of the disclosure.

In some embodiments, the method for producing a plant material comprises:
a) providing a plant of the *Brassica, Spinacia,* or *Lactuca* genus stably transformed to express the biologically active recombinant protein;
b) harvesting a plant material from the plant of step a).

In some embodiments, the method for producing a plant material further comprises the step(s) of:
c) drying the plant material, preferably by freeze-drying or lyophilization, and/or
d) homogenizing the plant material.

According to the present disclosure, steps (a) to (d) of the method according to the disclosure or as detailed herein may be, and preferably are performed in the following order, i.e. step (a) precedes step (b), which in its turn precedes step (c), which in its turn precedes step (d). It is however to be understood that the homogenization of the plant material carried out in step (d) may be performed before the drying the plant material in step (c).

In some embodiments, the method for producing a plant material comprises the step(s) of:
a) providing a plant of the Brassica, Spinacia, or Lactuca genus stably transformed to express the biologically active recombinant protein;
b) harvesting a plant material from the plant of step a), preferably leaves from the plant of step a),
c) drying the plant material, preferably by freeze-drying or lyophilization,
d) homogenizing the dried plant material.

The steps (a) to (d) are further described below.

### Step a) providing plant genetically modified

The plant expressing the biologically active recombinant protein may be obtained according to any method known in the art.

According to the disclosure, the plant is a biologically active recombinant protein producer.

The genetically modified plant comprises a non-native construct capable of expressing the biologically active recombinant protein in the plant.

In some embodiments, the plant genetically modified to express a biologically active protein is obtained carrying out the method as previously set out by the applicant and as described in WO 2023/036984 A1. Briefly, this method uses chemically synthesized nucleic acid construct (i.e., de novo synthesized). The applicant has previously shown that large single nucleic acid construct (> 2000 bp) can be used to stably transform a plant and allow to product multimeric proteins from a single nucleic acid construct (or expression vector), where a combination of vectors is classically used. The method for preparing the GM plant from which the plant material used in the present disclosure is briefly detailed below.

In some embodiments, the plant genetically modified to express a biologically active protein is obtained by a method for producing a genetically modified plant comprising a step of introducing in the plant a single nucleic acid construct that provides for stable expression of the biologically active protein into the plant.

In some embodiments, the plant genetically modified to express a biologically active protein is obtained by a method for producing a genetically modified plant expressing a protein of interest comprising a step of i) introducing in a plant a single nucleic acid construct that provides for stable expression of the biologically active protein into the plant; wherein:
- the protein of interest is the biologically active protein,
- the plant is selected from the *Brassica, Spinacia,* and *Lactuca,* and genus, preferably the plant is an edible plant from the *Brassica* genus.

Typically, the nucleic acid construct is preferably a single synthetic construct comprising a regulatory sequence active in a plant for the expression of an operably linked DNA, an operably linked protein-coding DNA molecule, and a 3' untranslated region.

Typically, the method for producing a genetically modified plant further comprises a step of ii) obtaining a transgenic plant comprising the DNA construct that stably expresses the protein of interest by regenerating the transgenic plant from the plant, plant cell, protoplast, or plant tissue that received the nucleic acid construct.

Typically, the method for producing a genetically modified plant uses a nucleic acid construct further comprising one or more of the following sequences: a 5' untranslated sequence, a signal sequence, an enhancer sequence, a cis-acting element, an intron sequence, a transcriptional Terminator Sequence (TTS), and one or more selectable marker coding sequences.

In some embodiments, the method for producing a genetically modified plant is a method wherein the protein of interest is a multimeric protein, and the protein-coding DNA molecule is a single synthetic molecule comprising a nucleic acid sequence coding for the different subunits of the multimeric protein.

In some embodiments, the method for producing a genetically modified plant is method wherein the protein of interest is an antibody and the protein-coding DNA molecule is a single synthetic molecule comprising a nucleic acid sequence coding for an antibody light chain or the antigen-binding fragment thereof and a nucleic acid sequence coding for an antibody heavy chain or the antigen-binding fragment thereof, optionally wherein the antibody is an anti-PD-1, optionally wherein the antibody is nivolumab.

The sequences mentioned herein are described in **Table 4.**

In some embodiments, the method for producing a genetically modified plant is a method wherein the protein of interest is nivolumab and the nucleic acid coding for the protein of interest comprises nucleic acid sequences coding for VL and VH sequences having at least, 80, 85, 90; 91, 92; 93; 94; 95; 96; 97; 98; 99 and 100 % identity respectively to the VL and VH sequences of SEQ ID NO:1 (Nivolumab VH sequence) and SEQ ID NO:2 (Nivolumab VL sequence) or the antigen binding fragment thereof, or with the CDRs portion thereof.

Several methods are available to determine identity between two sequences. A preferred method involves determine identity between two sequences through a BLAST search. BLAST (Basic Local Alignment Search Tool) is a set of algorithms and programs designed to find similarities between biological sequences e.g., DNA, RNA, or protein as described in Atschul et al, Journal of Molecular Biology, 215, 403-410, 1990. Preferably the default parameters of the algorithms and computer programs are used.

In some embodiments, the method for producing a genetically modified plant is a method wherein the multimeric protein-coding nucleic acid synthetic molecule comprises 2 tag sequences located at the 3' end of each monomer coding sequence, optionally wherein the antibody-coding DNA synthetic molecule comprises a Tag sequence at the 3' end of the light chain coding sequence and a Tag sequence at the 3' end of heavy chain coding sequence, optionally wherein the protein-coding nucleic acid molecule codes for a protein having at least 90 % identity with a sequence of SEQ ID NO: 3 (Nivolumab construct sequence) or has at least 60 % identity with the nucleic acid sequence of SEQ ID NO:4 (Optimized coding sequence for Nivolumab (minusTags)) or SEQ ID NO:5 (Non-optimized Nivolumab (minus Tags) coding sequence (nt)).

In some embodiments, the method for producing a genetically modified plant is a method wherein the nucleic acid construct is introduced into the plant or plant cells using (i) a direct DNA uptake method, or (ii) agrobacterium-mediated plant transformation, typically wherein the nucleic acid construct is inserted between the DNA border repeats of an agrobacterium-mediated plant transformation binary vector (T/DNA binary vector).

Direct DNA uptake methods and agrobacterium-mediated plant transformation methods are well known by the skilled person.

In some embodiments, the method for producing a genetically modified plant is a method further comprising the steps of i₁) preparing a transformant by introducing a nucleic acid construct into a bacterial strain; and i₂) transforming the plant, plant cell, protoplast, or plant tissue using the transformant.

In some embodiments, the method for producing a genetically modified plant is a method wherein the strain is an agrobacterium strain, notably an A. tumefaciens strain.

In some embodiments, the method for producing a genetically modified plant is a method wherein at step i1) the bacterial strain is obtained using a binary vector system, wherein the bacterial strain is co-transfected with a T DNA binary vector and a vic helper plasmid, or wherein a T DNA disarmed A. tumefaciens strain is transfected with a T/DNA binary vector.

In some preferred embodiments, the strain is typically an agrobacterium strain, notably an A. tumefaciens strain. This bacterial strain typically comprises (i) a Ti plasmid including a T-DNA region comprising or consisting of a synthetic expression cassette as previously defined or (ii) a binary vector system, wherein the binary vector comprises a T-DNA/RNA region comprising or consisting of a synthetic expression cassette as previously defined. In some embodiments the T-DNA region comprises a nucleic acid sequence having at least, 80, 85, 90; 91, 92; 93; 94; 95; 96; 97; 98; 99 and 100 % identity with SEQ ID NO:4 (Optimized coding sequence for Nivolumab (minusTags)), SEQ ID NO:5 (Non-optimized Nivolumab (minus Tags) coding sequence (nt)), SEQ ID NO:6 (Nucleic acid construct CaMV35S-Nivolumab (Optimized for Arabidopsis Thaliana but works in cabbage) +promoter, +tags), SEQ ID NO:7 (Nucleic acid construct CaMV35S-Nivolumab (original) (with promoter tags)) or SEQ ID NO:8 (Nucleic acid construct (Nos-nivolumab) with promoter and tags).

In some embodiments, the nucleic acid of interest codes for a protein, in particular for a multimeric protein, having a molecular weight comprised between 10 kDa and 170 kDa, between 10 kDa and 150 kDa, between 60 kDa and 170 kDa, or between 60 kDa and 150 kDa.

In some embodiments, the nucleic acid of interest codes for a multimeric protein having a molecular weight comprised between 20 kDa and 170 kDa, between 20 kDa and 150 kDa, between 60 kDa and 170 kDa, or between 60 kDa and 150 kDa.

In some embodiments, the nucleic acid of interest codes for a multimeric protein, for example an antibody fragment, having a molecular weight comprised between 20 and 130 kDa, between 25 and 130 kDa, between 20 and 100 kDa, between 25 and 100 kDa, between 20 and 70 kDa or between 25 and 70 kDa.

In some embodiments, the nucleic acid of interest codes for a multimeric protein, for example an antibody, having a molecular weight comprised between 130 and 170 kDa, preferably between 140 and 160 kDa, more preferably between 145 and 150 kDa.

In some embodiments, the antibody is a biosimilar antibody for a reference approved monoclonal antibody, notably a biosimilar for Nivolumab.

In some embodiments, the nucleic acid coding for the protein of interest comprises nucleic acid sequences coding for VL and VH sequences having at least, 80, 85, 90; 91, 92; 93; 94; 95; 96; 97; 98; 99 and 100 % identity respectively to the VL and VH sequences of SEQ ID NO:1 and 2 or the antigen binding fragment thereof, or with the CDRs portion thereof.

Although both VH and VL domains are naturally encoded by different gene, in the de novo synthesized coding nucleic acid of the present disclosure, the nucleic acid sequences coding for the VH and VL domains are typically fused to form a single synthetic nucleic acid, such that the whole antibody can be produced from a single nucleic acid construct.

In some embodiments, the nucleic acid coding for the protein of interest (to be produced) include one or more protein tags (also named herein molecular tags) usable for purification of the protein of interest from the plant (notably the plant cell or plant tissue(s) as defined previously). Typically, in embodiments, where a multimeric protein is produced according to a method of the present disclosure, the nucleic acid construct is synthesized such as a molecular tag is de novo attached to each nucleic acid sequence encoding for a given monomer (i.e., polypeptide chain of the multimeric protein), in particular a different molecular tag is attached to each "monomer-coding" nucleic acid sequence. Typically, the molecular tag is attached to the 3' end of said sequence(s).

For example, in embodiments, where the protein of interest is an antibody comprising a VH and a VL domain, a molecular tag is attached to each sequence coding respectively for the VH and VL sequences. Typically, the molecular tags are attached to the 3' end of the VH and VL coding sequences. Typically said molecular tags are different.

The use of tag attached to the nucleic acid coding sequence, and in particular attached to the coding sequence corresponding to each coding sequence of the multimeric protein, is highly advantageous as it may serve two critical functions. First these tags can be used to detect the expression of the recombinant protein. Indeed, the expression level of the protein of interest can easily be assessed using antibodies directed against the selected tag. Furthermore, it allows for double simultaneous purification of the various polypeptide chains of the recombinant multimeric protein. In particular in the case of a recombinant antibody, the use of tags attached to each of the coding sequence of the VH and VL domain allows for double simultaneous purification of the full antibody. This method of purification improves yield and efficiency of purification, while reducing waste and run off. In some embodiments, selected tags (like (poly)histidine tags) are also advantageous in affinity chromatography and in particular in immobilized metal ion affinity chromatography (IMAC). Having more than one tag is advantageous as the sample can be purified sequentially with each tag.

In some embodiments, the synthetic nucleic acid of interest comprises nucleic acid sequences coding for VL and VH sequences having respectively at least, 80, 85, 90; 91, 92; 93; 94; 95; 96; 97; 98; 99 and 100 % identity with SEQ ID NO:3, notably with the VL and VH sequences of SEQ ID NO:3.

In some embodiments, the synthetic nucleic acid of interest comprises a nucleic acid sequence coding for an amino acid sequence having at least, 80, 85, 90; 91, 92; 93; 94; 95; 96; 97; 98; 99 and 100 % identity with SEQ ID NO:3.

In some embodiments, the synthetic nucleic acid of interest comprises a nucleic acid sequence coding for an amino acid sequence having, 1; 2; 3; 4; 5; 6; 7; 8; 9; 10; 11; 12; 13; 14 or 15 amino acid substitution(s) as compared with SEQ ID NO:3.

In some embodiments, the synthetic nucleic acid of interest comprises a nucleic acid sequence having at least, 80, 85, 90; 91, 92; 93; 94; 95; 96; 97; 98; 99 and 100 % identity with SEQ ID NO:4 or 5.

### Step b) harvesting

The plant material is harvested from a plant of step a).

The step of harvesting plant material from a plant involves the collection of plant parts such as leaves, flowers, fruits, seeds, stems, or roots from the whole plant.

The skilled person knows the timing and techniques to harvest such plant parts.

In some embodiments, the plant material that is harvested is leaves.

In some embodiments, the leaves are harvested when they are mature but before they start to wither. In some embodiments, the leaves are harvested before flowering.

### Step c) drying

The plant material may be dried.

Such plant material may be a plant material harvested obtained according to step b).

The drying step involves the removing of water from the plant material. Preferably, the drying step is carried out in such a way as to break down plant tissues with little or no breaking of the cell walls of the plant cells.

The drying step is preferably carried out without heating. In some embodiment, the drying step is not carried out by drying in a hot air oven e.g. at temperatures 45-55°C.

The skilled person knows the techniques to dry a plant material, in particular to dry a plant material without heating. Drying may for example be carried out by drying, spray drying, freeze-drying, or lyophilization.

As used herein, "spray drying" refers to a drying a method involving transforming the plant material into a liquid or slurry which is then atomized into droplets and then quickly dried by hot air, producing a fine powder.

As used herein, "freeze-drying" refers to a drying a method involving freezing the plant material and then subjecting it to a vacuum, allowing the frozen water to sublimate directly from ice to vapor without passing through a liquid phase.

As used herein, "lyophilization" refers to a drying a method freezing the material and then removing the ice by sublimation under vacuum conditions.

Freeze-drying and lyophilization are preferred since they retain the structure and properties of the original material better than other drying methods.

Lyophilization is preferred.

### Step d) homogenization.

The plant material may be homogenized.

The plant material that is subjected to homogenization is preferably a dried plant material obtained according to step c).

A plant material homogenate is obtained.

The homogenization step involves breaking down and mixing plant tissues into a uniform and consistent sample. Preferably, the homogenization step is carried out in such a way as to break down plant tissues with little or no breaking of the cell walls of the plant cells.

The skilled person knows the techniques to homogenize a plant material. Homogenization may for example be carried out by bead beating, liquid nitrogen grinding or ultrasonication.

As used herein, the term "bead beating" refers to an homogenization technique involving the use of beads typically made of glass or metal along with a homogenization device.

As used herein, the term "liquid nitrogen grinding" refers to a homogenization technique involving freezing plant material using liquid nitrogen and then griding it to a fine powder.

### 3. Pharmaceutical composition

The plant material for use as provided in the first object of the present disclosure is preferably administered in the form of a pharmaceutical composition.

Thus, in a third object, the present disclosure relates to a pharmaceutical composition comprising a plant material as described in the first object of the disclosure, and an orally acceptable support.

The pharmaceutical composition is used a medicament as detailed in the first object of the disclosure.

In the context of the disclosure, the term "orally acceptable support" denotes substances such as excipients, carriers, adjuvants, buffers or the like which are suitable to be used, in combination with the plant material, for the preparation of an oral medicament.

Orally acceptable supports include fillers and bulking agents, e.g. lactose, microcrystalline cellulose), disintegrants (e.g. sodium starch glycolate, croscarmellose sodium), binders (e.g. starch, PVP, HPMC), lubricants (e.g. magnesium stearate), glidants (e.g. colloidal SiO₂), solvents/co-solvents (e.g. aqueous vehicle, propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), humectants (e.g. propylene, glycol, glycerol, sorbitol). Other suitable pharmaceutically acceptable supports are inter alia described in Remington's Pharmaceutical Sciences, 15th Ed., Mack Publishing Co., New Jersey (1991) and Bauer et ak, Pharmazeutische Technologic, 5th Ed., Govi-Verlag Frankfurt (1997). The person skilled in the art knows will readily be able to choose suitable orally acceptable supports, depending, e.g., on the formulation of the pharmaceutical composition.

The pharmaceutical composition to be administered orally may be formulated for example in the form of tablets, capsules, powders e.g. compact or loose powders, liquid suspensions or solutions.

In some embodiments, the pharmaceutical composition is in a dry form. The preferred oral form for the composition is a solid form such as a, a tablet, a capsule, a gum, or a powder (e.g. powder in sachet form).

In some embodiments, the pharmaceutical composition is formulated as a tablet. Such tablet may comprise excipients and other ingredients in suitable amounts. Such tablet may contain one or more of a binder (e.g. starch, cellulose derivatives such as hydroxypropyl cellulose, polyvinylpyrrolidone), a filler (e.g. lactose, microcrystalline cellulose, mannitol, dibasic calcium phosphate), or other excipients such as disintegrants or lubricants.

In some embodiments, the pharmaceutical composition is formulated as a capsule, the plant material being, for example, introduced into a shell consisting of a membrane of a coating material. The polymers forming the coating material may be of natural origin (gelatin, chitosan, etc.), semisynthetic origin (cellulose derivatives, etc.) or synthetic origin, such as the lactic and glycolic acid copolymers commonly used.

In some embodiments, the pharmaceutical composition is formulated as a gum, the plant material being embedded withing a gum. Such gum may comprise excipients and other ingredients in suitable amounts. Such gum may contain one or more of a stabilizer (e.g. polyvinylpyrrolidone (PVP), polyethylene glycol (PEG), cellulose derivatives such as methylcellulose or carboxymethylcellulose), a thickener (e.g. guar gum, xanthan gum, or synthetic polymers such as hydroxypropyl methylcellulose), and a plasticizer (e.g. glycerin, sorbitol, propylene glycol).

In some embodiments, the pharmaceutical composition is formulated as a powder, the plant material being mixed with excipients in suitable amounts. Such powder formulation may contain one or more of a bulking agent (e.g. mannitol, sucrose, trehalose, lactose), a stabilizer (hydroxypropyl methylcellulose, dextran, and cyclodextrins), or other excipients such as a surfactant or an antioxidant.

The pharmaceutical composition may be prepared according to any of the techniques known in the art, for example by mixing the plant material, and or more orally acceptable support.

The orally acceptable support can also be a food product such as a beverage, a drink, a food supplement, or a nutraceutical or an animal feed product.

The pharmaceutical composition preferably comprises the biologically active protein contained in the plant material as an active agent.

The pharmaceutical composition may comprise the biologically active recombinant protein contained in the plant material as the sole active agent, i.e. the biologically active recombinant protein for use in monotherapy.

Alternatively, the biologically active recombinant protein may be used as an adjunct to, or in combination with, one or more additional active agents, i.e. the biologically active recombinant protein may be used in combination therapy.

In some embodiments, two or more different plant materials are combined in a same pharmaceutical composition, for example in a same capsule, powder or tablet, to provide a combination therapy.

In some embodiments, the pharmaceutical composition is prepared by mixing two or more different plant materials as described in the first object of the disclosure, to provide a combination therapy.

In those embodiments, the pharmaceutical composition may comprise two or more biologically active recombinant protein contained in the two or more different plant materials as the active agents, i.e. the biologically active recombinant proteins for use in combination therapy.

In some embodiments, the pharmaceutical composition is formulated to deliver the biologically active protein to the GI tract, preferably by oral administration.

Other aspects and advantages of the present disclosure will emerge upon reading the examples which follow, which should be considered to be non-limiting illustrations.

### Legends of the figures

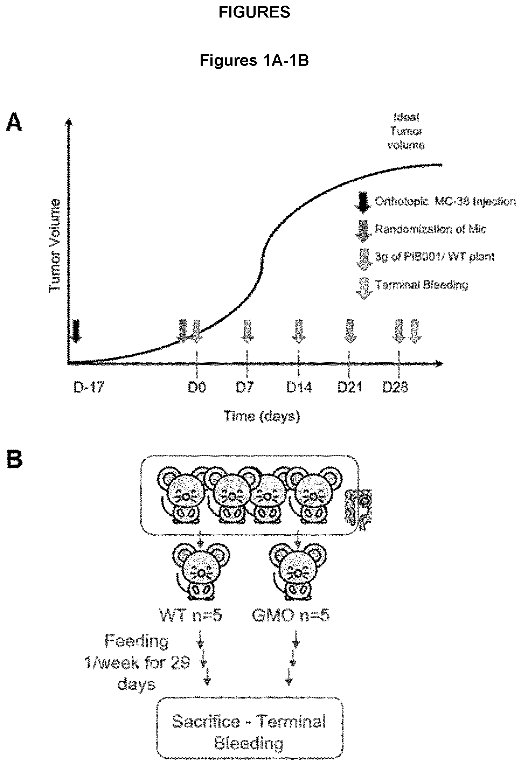

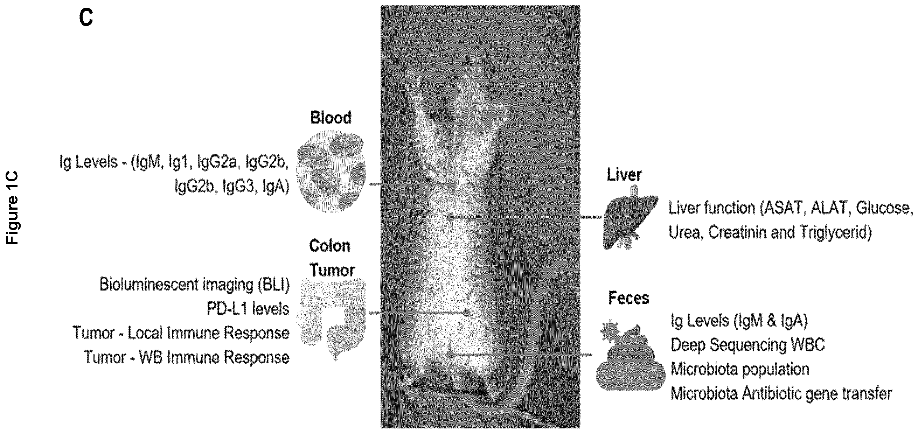

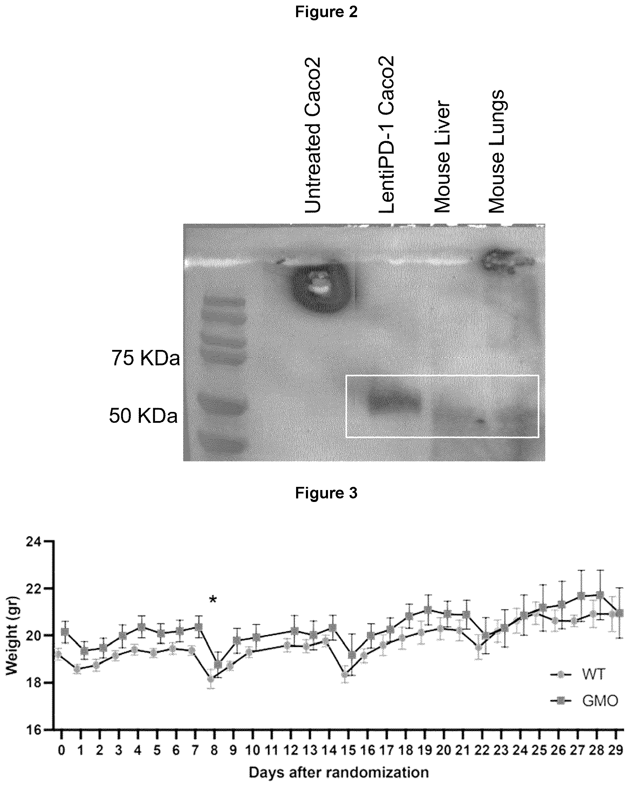

**Figure 1****. Overview of the study design of Example 1.**
   Orthotopic injection of MC38-Luc cells at D-17 in C57BI/6 mice of 6-7 weeks of age and tumor growth was monitored via BLI every 7 days on D0, D7, D14 and D21 (Figure 1A). One group received wild-type (WT) plant material, the other group received genetically modified (GMO) plant materia (Figure 1B) Outputs oth the study : Blood Ig levels (IgM, IgG1, IgG2a, IgG2b, IgG2b, IgG3, IgA), Liver function (ASAT, ALAT, Glucose, Urea, Creatinin and Triglycerid,; Feces - IgM & IgA - Weekly; Feces (-Deep Sequencing Microbiota population, -Deep Sequencing Microbiota Antibiotic gene transfer), Tumor (-Tumor growth (BLI), PD-L1 levels, -Local Immune Response, - WB Immune Response) (Figure 1C).
**Figure 2****. Western blot of murine PD-1 with Nivolumab.**
   The western blot analysis demonstrate that human Nivolumab is able to recognize mouse PD-1 receptor. 50 kDa bands (box) in positive control, mouse liver, and lung samples reveal recognition of murine PD-1 by human Nivolumab.
**Figure 3****. Mice (gr) weight over time.**
   Overview of the mean mice weights per treatment group (WT or GMO) during the study. Test items were administered at D0, D7, D14, D21, D28. A drop in mean weight can be observed the first day after every dosing. The GMO treated group demonstrated that the GMO plant did not have any adverse or toxic effects, which would result in significant weight loss over the next days. Damaged or effected small intestine and colon would result in weight loss. For the duration of the experiment the two groups continued to gain weight.

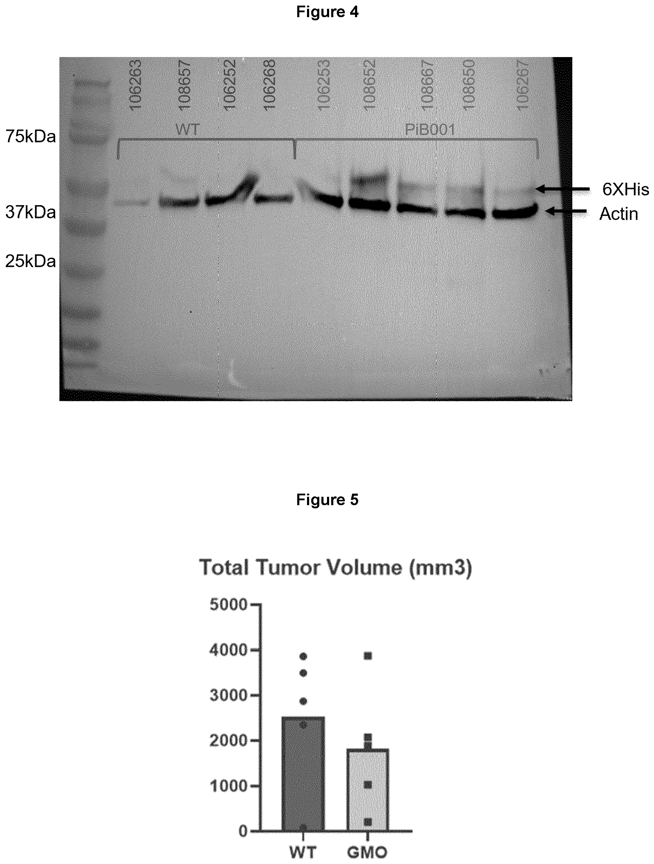

**Figure 4****. PiB001 in MC-38 draft at D29.**
   Dissected MC38 tumors from control and treated mice reveal presence of PiB001 in four out of five treated groups. Interestingly only mice with growing tumors showed the presence of PiB001 in them. PiB001 was detected using the 6xHis tag. Moreover, a band at 149kDa as well as multiple bands between 10-15 kDa were also observed (data not shown). These bands could imply protease cleavage of PiB001. However, enough of PiB001 reaches the site of MC-38 colon tumor draft to be detected and induce an immune response.
**Figure 5****. Tumor volume at D29.**
   Twenty-nine days after randomization, mice were sacrificed and tumor lesions were assessed by means of caliper measurements. The average tumor volume is lower in the GMO treated group. The variation observed arises because a tumor did not grow in one mouse (WT group), while one tumor in GMO group grew rapidly (GMO group).

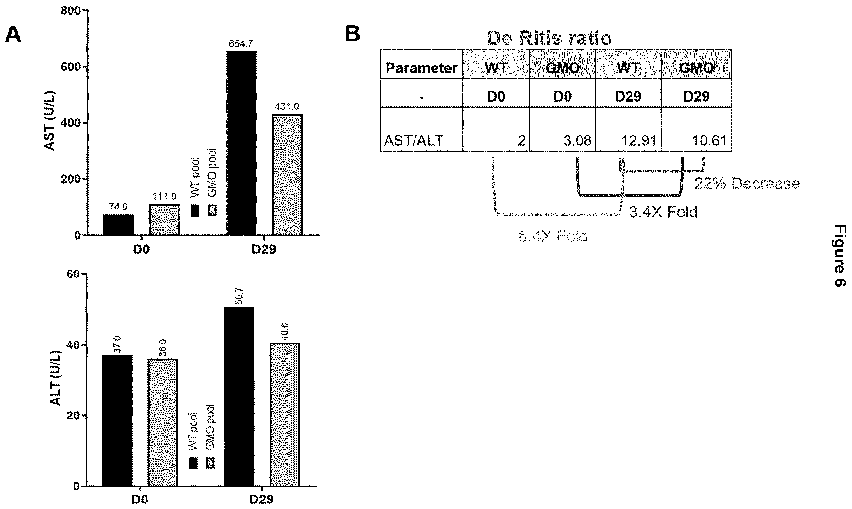

**Figure 6****. Colon AST & ALT (Pooled Samples) D0 & D29.**
   (**Figure 6A**) The graphs shows serum levels of aspartate transaminase (AST) and alanine transaminase (ALT) at D0 and D29. At D0 the levels of these enzymes are similar between the two groups. However at D29, AST and ALT serum levels in PiB001 treated group is lower than wild type plant treated group. This indicates that PiB001 group may have a protective effect compared to wild type plant treated mice because their colons are relatively healthier. (**Figure 6B**) De Ritis ratio between the two groups indicates that mice treated with PiB001 plant had 1.84x reduction in AST/ALT ratio between time points D0 and D29 when compared to mice treated with wild type plant. In addition these mice displayed a 22% decrease in AST/ALT ratio when compared to wild type plant fed mice.

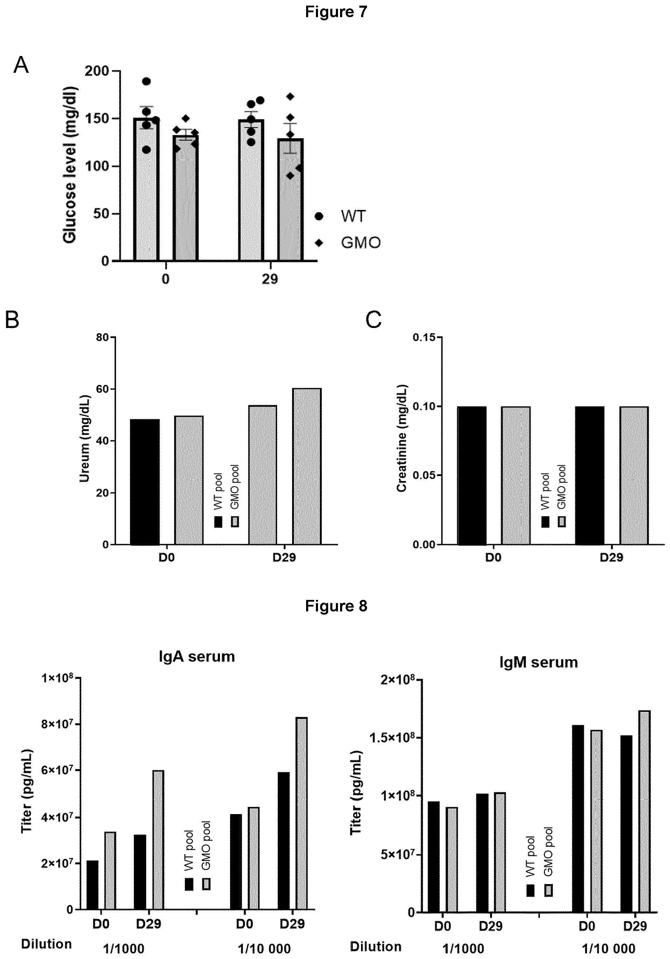

**Figure 7****. Glucose, Ureum & Creatinine serum levels (Pooled Samples) D0 & D29.**
   The graphs shows serum concentration of glucose (**Figure 7A**), ureum (**Figure 7B**), and creatinine (**Figure 7C**) at D0 and D29. Glycogenesis pathway in the livers is intact because excess glucose is being removed from the serum. Similarly the kidneys are also unaffected because they are removing ureum and creatinine from blood. This shows that the PiB001 does not have adverse effects on liver and kidney functions. Taken together the data shows the mice have a healthy metabolism.
**Figure 8****. Serum IgA & IgM levels (Pooled Samples) D0 & D29**
   The graphs show IgA & IgM titers in the serum. At day 29, PiB001 treated mice group displayed twice the serum titer compared to WT plant treated group. This indicates that PiB001 was able to stimulate intestinal immune system to secrete IgA. This IgA was of serum type which is detectable in the blood. However, no significant increase was observed in the serum IgM levels.

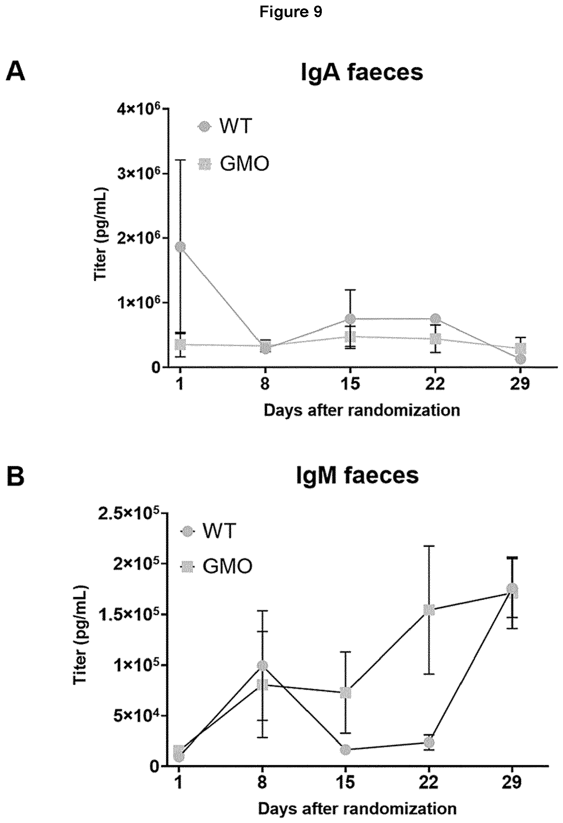

**Figure 9****. Feces IgA & IgM Concentrations.**
   The graphs display fecal titers of IgM and IgA antibodies. (Figure 9A) No significant increase was observed in fecal IgA levels between the two groups over the course of the treatment. (Figure 9B) The graph shows the measured response of IgMs for the duration of the experiment. The IgM titers continued to increases from day 8 onwards. For the course of the experiment IgM level continued to rise with continued treatments. At day 22, IgM titer was 6.5 times higher than the control. A steady rise in IgM titer indicates that PiB001 antibody induced an IgM response from interstitial immune cells.

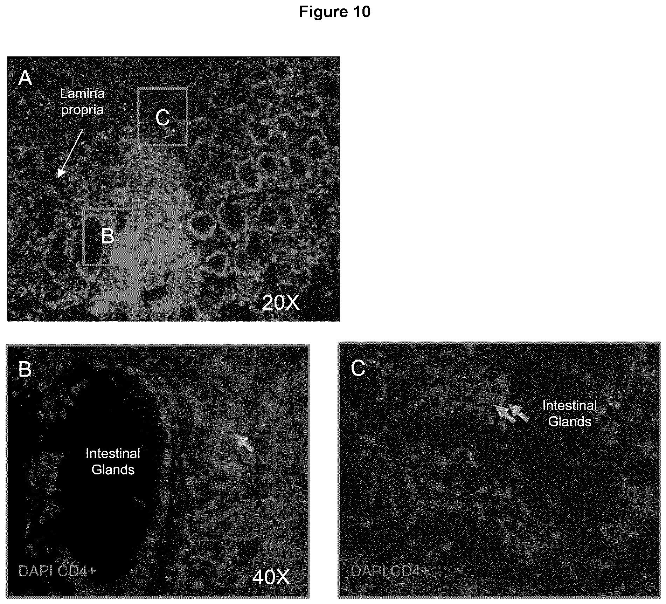

**Figure 10****. IHC of Normal Human Colon revealing CD4+ cells in the Colon.**
   The images show immunohistochemistry (IHC) on sections of healthy human colon. (**Figure 10A**) At 20x magnification of the mucosa near the lumen reveals, the presence of intestinal glands, lamina proper, and a cluster of T-cells. (**Figure 10B**) 40X magnification of this cluster shows CD4+ T-lymphocytes (arrow) in the tissue. (**Figure 10C**) CD4+ cells are near the lumen of the colon as well as in the submucosa. These CD4+ cells are capable of recognizing nivolumab to induce an immune response locally in the intestine.

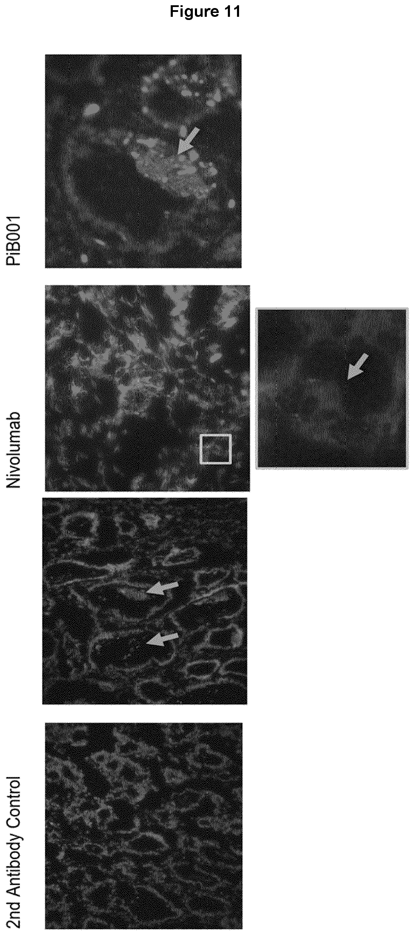

**Figure 11****. IHC on human Colon with adenocarcinoma comparing binding of Nivolumab and PiB001.**
   The images show immunohistochemistry (IHC) on human colons with adenocarcinoma. Staining with Nivolumab reveals presence of PD-1 positive cells with in the adenocarcinoma. Interesting Nivolumab stain adenocarcinoma within the intestinal glands. PiB001 staining reveals a similar to Nivolumab as it staging cells within the adenocarcinoma.

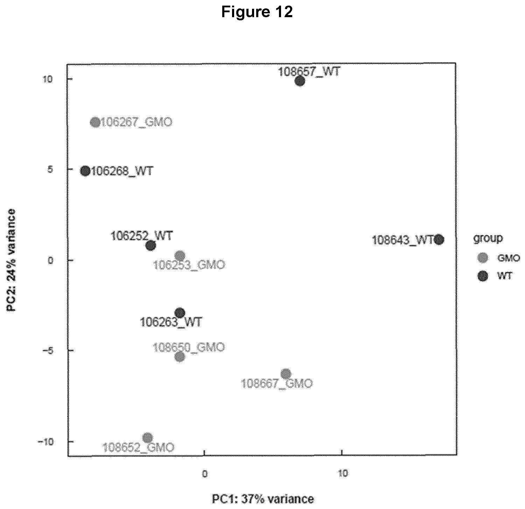

**Figure 12****. The graph displays principal component analysis based on RNA-seq of dissected MC-38 tumors.**
   The analysis shows undistinguished clustering between the two arms of the experiment. This means the RNA reads between the two arms was similar. This indicates that mice treated with PiB001 did not develop any excessive mutagenic characteristics as compared to tumors treated with wild-type plants.

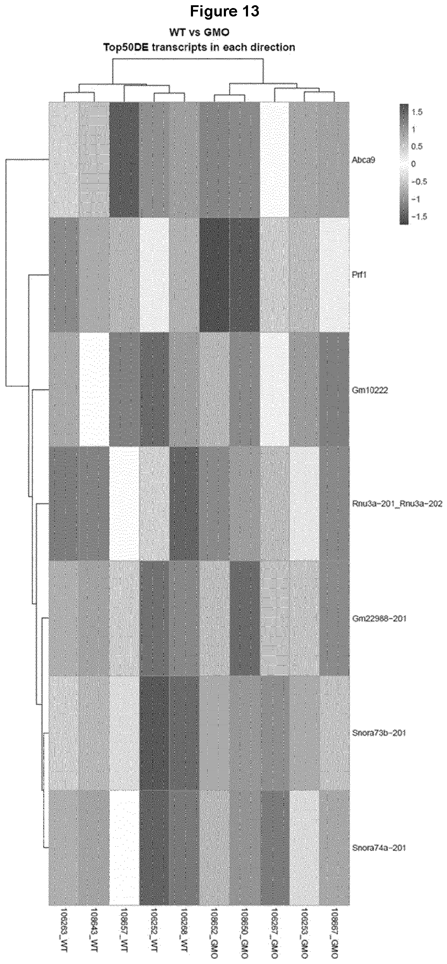

**Figure 13****. Heatmap displaying differential gene expression between tumors treated with wild-type plants and PiB001.**
   The heat map seven differentially expressed features with a minimum log2 fold-change of 1 at a significance level of P-adj <0.01 between the WT and PiB001 treated mice. The analysis concluded that the Abca9 and Prf1 gene was overexpressed in 4 out of the 5 mice treated with PiB001. These 4 mice were the same as those in which PiB001 was detected by WB (cf. **Figure 3**). Interestingly Prf1 gene is involved in production of perforin protein in immune cells (lymphocytes and natural killer cells). This protein forms membrane pores that allow the release of granzymes and subsequent cytolysis of target cells (in this case MC-38 tumor).

### EXAMPLES

### Example 1: Efficacy of a monoclonal plant-derived antibody in a CDX orthotopic colorectal cancer model using MC38-Luc cells.

### Compliance with Good Laboratory Practice (GLP)

This study was performed according to the standard operating procedures and methods described by ISO 9001:2015 and when applicable the Association for Assessment and Accreditation of Laboratory Animal Care (AAALAC). Good Laboratory Practice (GLP) was not applicable for this study.

### Ethical approval

This study was approved by the Animal Experimentation Committee (EC MxCI 2021-184). There are no generally accepted non-animal alternatives and the study does not unnecessarily duplicate previous experiments. Mouse and rats are rodent species commonly used for conducting research in pharmacology and toxicology. These species are readily available from reliable and trusted sources keeping detailed breeding and health records.

All procedures and methods of euthanasia that were used during this study were considered to avoid or minimize discomfort, distress, and pain to the animals. Animals experiencing severe or chronic distress or pain during this study, that could not be relieved, were euthanized after consultation of the animal welfare officer. However, animals that were moribund or in a state of impending death were humanely killed immediately as stated in the study plan.

### Study design

For this experiment, female C57BI/6 mice (n=25) of 6-7 weeks of age were used. At D-17 of the study, MC38-Luc cells were orthotopically injected in the caecum of each mouse. Mice were randomized at D0 via bioluminescent imaging (BLI) to obtain a total number of 5 mice/group for the entire study run. The additional 15 mice remaining were considered as spare animals to ensure proper randomization and the possibility to exclude significant outliers prior to the start of the treatment.

One group received wild-type (WT) plant material, the other group received genetically modified (GMO) plant material. The plant-derived monoclonal antibodies were subsequently administered by mixing it with Nutella. Mice were single housed overnight to ensure proper dosing. Blood was collected at D0 and D29 to evaluate antibody (IgM, Ig1, IgG2a, IgG2b, IgG3, IgA) levels, PD-L1 levels and liver function (AST, ALT, Glucose, Urea, Creatinine, and Triglycerides). Plant material was fed repeatedly the day of randomization (D0) and after 7, 14, 21, and 28 days. Tumor growth was monitored via BLI and was performed every 7 days on D0, D7, D14 and D21. Feces were collected additionally to analyze IgM and IgA antibodies with the use of a meso scale discovery (MSD). Feces collection was performed the day after plant dosing/feeding of the test item on D1, D8, D22 and D29.

All mice were sacrificed on D29 by terminal blood collection.

An overview of the study design is represented in **Figure 1A, Figure 1B** and **Figure 1C****.**

### Test item and control

The humoral immune response upon monoclonal plant-derived antibody treatment was assessed after weekly dosing of the following test item:
- Wild type plant material (control)
- GMO plant material comprising monoclonal plant-derived nivolumab (PiB001)

The GMO plant material used for dosing (test item) in this study was defined as:
- Molecular weight of PiB001 : 143-150 kDa
- Structural formula: Antibody in lyophilized plant material
- Appearance of plant material: Green, lyophilized powder
- Formulation buffer: NA
- Antibody concentration: 3-5 mg/g fresh leaf
- Manufacturer: Plantibodies SAS
- Endotoxins (LAL): NA
- Osmolality: NA

### Test item

The test item is a material comprising the plant produced monoclonal antibody against human PD-1.

The plants used in this manufacturing process are plants from the *Brassica* family, namely *Brassica* oleracea, *Brassica* oleracea var. capitata genetically modified to express nivolumab antibody following procedures as described in WO 2023/036984 A1.

Fresh transformed leaves from the plant are taken and placed in a mortar & pestle, followed by liquid nitrogen treatment. This process is known as lyophilization.

The plant material is instantly freeze-dried by sublimating the water content from the leaves. Then the leaves are gently grinded into a fine powder. The fine powder of the plant leaves is stored at -80°C until use.

### Test item preparation and storage

The plant-derived materials were handled as follows:
- Stored at -80°C.

Monoclonal plant-derived antibody was handled as follows:
- Take out the plant-material the day of dosing. Thaw it in the fridge for 1 hour prior to compound preparation (see below).

The formulations were prepared daily (day of administration/feeding), one hour prior to dosing, as described below:
- 3 gr of plant material was taken and put in a sterile tray.
- 0.6 gr of Nutella was added per 3 gr of plant material and mixed with a disposable spatula.
- Left-over and not-used plant material was stored at -80°C.
- Plant material exposure to the air was minimized whilst preparation.

### Test item dosing

Animals were dosed once a week with one of the test items.

Prior to dosing, mice were single housed (1h before dosing). Cages were foreseen with cage enrichment and water, however no additional food was provided to ensure fasting prior to test item feeding. A tray of plant-derived (WT or GMO) material mixed with Nutella was placed in the cages one hour later. Mice were able to eat overnight and were placed back in their original cages the consecutive morning (group housing).

### Treatment conditions

The treatment conditions are summarized in the **Table 1** below:

**Table 1: Treatment conditions.**

| Nr | Test item | Route | Volume | Dosing | N° animals |
|---|---|---|---|---|---|
| 1 | Wild type plant (WT plant-derived material) | Oral/feeding | 3gr/mouse | Weekly for 29 days | 5 |
| 2 | Monoclonal plant-derived antibody (GMO plant-derived material) | Oral/feeding | 3gr/mouse | Weekly for 29 days | 5 |

### Nivolumab binding to murine PD1

Immunoblotting based tests were carried out to test if Nivolumab, a fully humanized monoclonal antibody produced in mammalian cells such as HEK293 was able to recognize murine PD-1 receptor. It was hypothesized that PD-1 domains recognized by Nivolumab were similar or conserved between human and murine. Hence, using a humanized antibody in mice, might result bind of Nivolumab to murine PD-1

### Material and methods

Murine liver and lung tissues from wild type C57/BI6 mice were lysed in 1x RIPA for 1 hour on ice. The samples were centrifuged at 16000g for 10 minutes at 4C. In a fresh eppendorf tube 1X laemmli and DTT were added to the supernatant from lysed tissue, followed by addition of loading buffer. The samples were heated at 95°C for 10 minutes before loading on a precast 10% midi protein gel electrophoresis SDS gel (Biorad). The membrane were prodded with human recombinant Nivolumab (Thermofischer MA5-41974) and signal was detected using a HRP antibody. Caco2 cell line overexpressing human PD-1 was used as a positive control and untreated Caco2 cell line as a negative control.

### Results

The western blot analysis demonstrate that human Nivolumab is able to recognize mouse PD-1 receptor. 50 kDa bands in positive control (lentiPD-1, Caco2 cells over expressing hPD-1), mouse liver, and lung samples reveal recognition of murine PD-1 by human Nivolumab. This data shows that administration of human nivolumab to WT mice would allow it to recognize and bind to murine PD-1. Hence orally administration of PiB001 to mice allows nivolumab to bind to murine PD-1 and induce an immune response.

### Animals

6-7 week-old female C57BL6/NCrl mice (Charles River) with SPF health status were used for this experiment. For this study, 14 mice (n= 7/group) were included in the study after randomization to ensure a group size of 5 mice/group through the entire study run. Fifteen spare animals were ordered to ensure the most optimal experimental setup. Group housing was standardly applied to guarantee normal animal behavior. However, individual housing was applied for dosing or indicated for medical reasons, for close observation or to prevent social aggression. The decision to do so could be taken by animal caretakers, biotechnicians and veterinarians but needed to be confirmed by a veterinarian within 3 days after initiation.

### Animal coding

All animals received a unique animal ID number. These ID numbers were visualized on cards attached to each cage containing the corresponding animals. Animals received a unique ear tag upon the start of the experiment. These ear tags were coupled with the unique ID number to enable individual follow-up.

### Experimental groups

The animals were allocated to different experimental groups depending on treatment conditions. After allocation to the groups, the animal numbers were complemented by a code containing the dosing group identification.

The animals were subdivided into different experimental groups depending on randomization at D0 to the treatment conditions.

The experimental conditions applied after randomization (D0, initial group sizes of 7 mice/group) are summarized in the following **Table 2:**

**Table 2: Overview of the different experimental groups in the study.**

| Cage | Group | Test item | Mice (N) | Procedures |
|---|---|---|---|---|
| 1 | A | Wild type plant (WT plant-derived material) | 3 | Feeding: once a week starting from D0 to D28 |
| 2 | A | Wild type plant (WT plant-derived material) | 4 | BLI: once a week starting from D0 to D21 |
| 3 | B | Monoclonal plant-derived antibody (GMO plant-derived material) | 4 | Feces collection: once a week starting from D1 to D29 |
| | | | | Blood collection: D0 and D29 |
| 4 | B | Monoclonal plant-derived antibody (GMO plant-derived material) | 3 | |

### Animal allocation

Prior to allocation, at the discretion of the study director, animals with abnormalities were likely to be prejudicial to the study and were therefore excluded from allocation. If animals were excluded from allocation, the sponsor was notified before the start of the study. Animals were allocated to an experimental group before the start of the study.

### Microbiological monitoring

Upon arrival of the animals, a FELASA health check was performed on the used batch of animals by sending samples and/or live animals to an external diagnostic laboratory. Conclusions concerning microbiological health of animals are archived. As the study did not continue for a period of 3 months, no sentinels were included in the study setup and no quarterly screening was performed. In case of anomalies, results were reported to the sponsor within 24h. This was not the case for these mice.

### Accommodation and environmental control

After arrival, animals were allowed to acclimatize for 2 weeks before the start of the experiments. Hereafter and before the start of the experiment, animals were moved from quarantaine to the experimental unit. Animals were housed in individually ventilated cages (IVCs) with sterilized, dust free bedding. Cages (Innovive, USA) were provided with cage enrichment to stimulate natural patterns of behaviour. Animals were maintained at a range between 20-24°C and a relative humidity between 40 and 70% on a 12/12 light/dark cycle with air renewal of 8-20 times per hour. These parameters were checked and recorded. Quality control of bedding was performed regularly at vendor and the documentation can be requested. No interaction was expected between the test items and the usual constituents of bedding.

### Diet and water supply

The animals had free access to standard pelleted food (diet No. V1534-703, SNIFF, Germany) and water in Aquavive drinking bottles (Innovive). Quality control of diet and water was performed regularly at vendor and the documentation can be requested. No interaction was expected between the test compound and the usual constituents and contaminants of diet and water. Food pellets and gelled water were provided at the bottom of the cage in need to ensure proper access to food and water, hence ensuring proper wellbeing.

### Experimental procedures and parameters

The experimental procedures and parameters applied for this study are described below.

### MC38-Luc cell culture

Cells were thawed 2 weeks before the start of the study. MC38-Luc cells (cat no. 78383, BPS Bioscience) were cultured in DMEM medium (cat no. 41966-029, ThermoFisher Scientific), supplemented with 10% heat-inactivated fetal bovine serum (FBS, cat no. S181B-500, Biowest) and 1% penicillin/streptomycin at 37°C with 5% CO₂ in a humidified incubator. Every 2-3 days, medium change was conducted and/or cells were split at a 1:4 to 1:10 ratio.

Cells were expanded until sufficient cell numbers were reached for the experiment. Before injection, cells were put a minimum of 1 passage without penicillin/streptomycin. At the day of injection, heat-inactivated FBS was eliminated from the medium through washing steps and cells were collected on ice in DMEM medium. Cells (passage 27, 0.5 x 106 cells) were injected in a volume of 40 µl pure sterile DMEM.

### Orthotopic injection of MC38-Luc cells

Seventeen days before the start of the study, mice were anesthetized with (2-3%) isoflurane, followed by analgesia through the subcutaneous (SC) administration of carprofen (5 mg/kg). An incubation time of 5-10 minutes was foreseen to ensure proper efficacy of the analgesia. Subsequently, the abdomen of each mouse was shaved and disinfected with a 70% ethanol solution. A first incision was made in the skin of the abdomen, followed by a second incision opening the peritoneum. Both incisions were performed with different material to maintain sterility. A sterile gauze (cat no. 2800218, Covetrus) was placed on the abdomen to ensure a sterile working field. The caecum was placed on the gauze and was hydrated with a saline solution prior to injection. Cells were then injected into the caecum in a volume of 40 µl with an insulin syringe (U-100 insulin syringe, 0.33 mm - 29G needle, BD). Next, the caecum was rinsed externally with a cotton swab (cat no. 2800165, Covetrus) drenched in a saline solution to remove any external cellular remnants. After inoculation, the peritoneum and the skin were sutured individually with absorbable vicryl sutures (cat no. JV389, Ethicon). Mice were checked regularly after surgery and daily analgesia was applied SC for a duration of 7 days.

### Follow-up

Animals were observed daily (from D0 onwards) to evaluate the behavior of the mice. Food and water was checked, and general behavior was noted down. If mice did not show an optimal behavior, color coded tags on the cage indicated that extra care was needed. The Study Director as well as the Designated Veterinarian were informed about this behavioral change. The frequency of observation in a day was adjusted to the manifestation of clinical signs or discomfort of the mice. During the initial 24 hours after injection, animals were observed frequently for any behavioral and physical abnormalities. In case animals appear in severe discomfort, animals were humanely killed followed by necropsy. The circumstances of severe discomfort, deaths or intermittent terminations were recorded and reported to the sponsor within 24 hours. To monitor disease progression, a standard scoring system was used. In addition, food intake was monitored by registered weighing. This was performed daily throughout the entire time span of the study as an indicator for well being.

A standard scoring system is detailed in the **Table 3** below:

**Table 3: Standard Scoring system**

| | Parameter | Observations | Score |
|---|---|---|---|
| **A.** | Visual aspect | Normal | 0 |
| | | Decreased grooming | 1 |
| | | Piloerection (ruffled fur) | 2 |
| | | Piloerection, nasal and eye discharge or hunched posture | 3 |
| | | Piloerection, hunched posture and semi-closed or clsed eyes; drawn facies | 3 |
| **B.** | General clinical signs | Normal breathing rate | 0 |
| | | Increased breathing rate and cyanosis (blue skin) and abdominal breathing | 2 |
| | | Marked abdominal breathing rate and cyanosis (blue skin) AND/OR oedema of upper respiratory tract (swelling neck) | 4 |
| | | Dehydration (skin fold test) | 2-3 |
| | | Anemia (symptoms : pale ears and feet) | 3 |
| | | Body weight loss >20% from the maximum weight measured on the animal | 4 |
| | | Ascites with marked abdominal enlargement | 4 |
| **C.** | Spontaneous behavior | Normal | 0 |
| | | Minor changes | 1 |
| | | Decreased mobility, seclusion, alert behavior | 2 |
| | | Placid behavior, immobility | 3 |
| | | Vocalizations, automutilation or immobility > 24h | 4 |
| **D.** | Provoked behavior | Normal | 0 |
| | | Slight depression or exaggerated behavior to induced stimuli | 1 |
| | | Moderate changes of induced behavior | 2 |
| | | General weakness/no response | 4 |
| **E.** | Nervous symptoms | Circling, convulsions | 4 |
| *The severity is dependent of the extent and of the localization of the observed reaction* | | | |

### Score information:

- if the score is 3 or +, more than one time, add 1 supplement point for each "3" score
- Each individual "4" score has to be considered as a decision point for euthanasia.

### Assessment of the global score, including all categories, for a total between:

- 0-5 = Normal
- 6-9 = Attentive care, consider analgesics use or possible treatment after discussion with the vetenary responsible for animal welfare
- 10-14 = Proven suffering, closed care, consider euthanasia
- 15 and more = severe pain/discomfort. Euthanasia and necessity to reconsider the experimental protocol with the ethical committee. Randomization

At the start of the experiment (D0), tumor growth was measured through BLI. Mice were then randomized based on the signal intensity of the BLI images. This was done in such a way that the mean signal intensity of the two treatment groups (vehicle/WT control and plant-derived monoclonal antibody/GMO) were as similar as possible.

### Bioluminescence imaging (BLI)

BLI was performed 0, 7, 14, and 21* days after the start of the study (D0). To do so, luciferin (15 mg/ml, cat no. 12299, Perkin Elmer) was injected SC in a concentration of 126 mg/kg, and a consequent imaging was performed 10 min later (* see deviation section). Images were taken from mice placed on their back (so that their belly is visible in the picture) while anesthetized with (2-3%) isoflurane. Exposure time was kept 1 sec, 30 sec, and 60 sec for each mouse/picture over time during the experiment to allow optimal imaging during the entire experiment.

For the analysis of the BLI images, pictures with an exposure of 60 sec were used for all timepoints and groups to standardize the raw data based on exposure time. In addition, all images were put on the same scale to mediate correct data analysis. Signal intensity (Radiance) was assessed by applying a standard region of interest (ROI) with the same size over the abdominal surface of the mice. Both total flux; photons/sec (p/s) and average radiance (p/s/cm2) were used to compare tumor growth between groups.

### Feces collection

Feces collection was performed 1, 8, 15, 22, and 29 days after randomization. Vials were labeled in advance with the corresponding sample ID, treatment group and date. Each mouse was then placed individually in an empty cage without bedding. The first four fecal pellets were collected in the corresponding vial with the use of a sterile toothpick or forceps (2 fecal pellets per vial). All vials were transferred to ice. One vial was stored at -80°C freezer while the other vial containing fecal samples was stored in a -80°C freezer after processing. Samples were always collected in the morning to avoid large deviations.

Fecal sample processing (1 of the 2 vials per mouse per time point) was done on freshly isolated fecal samples. The processing included the addition of 100 ml extraction buffer (PBS containing 0.05 % Tween, 0.5% FBS, 1mg/ml EDTA, 1 mg/ml phenylmethylsulfonyl fluoride, and 200 mg/ml trypsin soybean inhibitor) per mg feces. The samples were incubated for 15 min on ice thereafter they were homogenized whilst vortexing. Next the samples were centrifuged at 23,000 x g for 15 min at 4°C. The supernatant was stored at -80°C until use.

### Glucose measurement

To measure the amount of circulating glucose, mice were fasted for 2 hours prior to blood sampling on D0 and D29. One drop of whole blood was collected and captured on the GlucoMen Areo Sensor strip. Glucose was measured with the GlucoMen Areo.

### Blood sampling

An intermediate blood collection was conducted at the start of the study (D0). Vials were labeled in advance with the corresponding sample ID, treatment group and date. Blood was collected from the mandibular vein. The blood collection volume was based upon the body weight of each mouse. This blood was pooled per group and transferred to an EDTA tube. Leftover blood per group was pooled and transferred to a serum tube to determine antibody and PD-L1 levels (Sarstedt). In both cases, this was done after discarding the needle to prevent extra disrupting of blood cells.

The EDTA tubes were stored at room temperature until transport to Zoolyx NV for the determination of liver function.

The serum tubes were stored overnight at 2- 8°C. Afterwards the supernatant was transferred to a new collection tube and centrifuged 10 min at 3400 g at 4°C. The top layer, being the serum, was transferred to a new tube, clearly labeled, and stored at -80°C. Any comments like the serum volume and appearance of haemolysis were noted. For this procedure IS-03-370 work instructions were followed.

### End of experiment

The experimental procedures that were applicable at the end of the study are described below.

### Euthanasia

Animals were followed-up for a maximal study duration of 29 days after the start of the study (D0; randomization). Follow-up could be shorter if humane endpoints were reached or upon request of the sponsor, but this was not necessary. Terminal bleeding was performed through cardiac puncture. Mice were then killed humanely by cervical dislocation (Cfr. IS-04-371).

A humane endpoint would be carried out when animals show changes in physical appearance (e.g., coat texture; hair soiled with urine or feces); changes in clinical signs (e.g., dyspnoea; posture; diarrhea for more than 2 days); >15% of body weight loss in a period of less than a week or chronic weight loss > 20% in the last 3 weeks; changes in food and water consumption, presence of persistent and non-treated infection; changes in unprovoked behavior (e.g., self-mutilation; compulsive behavior; posture; grooming patterns; activity levels); behavioral changes in response to external stimuli (e.g., excitability; righting reflex) or when indicated by the score.

After any procedure, animals were monitored closely for 1-hour post-intervention and then on a daily basis. If animals appear weaker or immobile immediately after intervention, solid water in jelly and food pellets will be put next to the mice for easy access to a water and food source until they recover. Any animals observed to be in poor health or pain will be identified for further monitoring, pain medication and possible euthanasia.

### Terminal bleeding

Terminal bleeding occurred through cardiac puncture under anesthesia (3% isofluorane). Whole blood was collected in a in a 1 ml syringe with 26G needle (BD). This blood was then transferred to either an EDTA tube or a serum tube (pooled per group) for larger volumes (Sarstedt). This was done after discarding the needle to prevent haemolysis.

EDTA tubes were kept at room temperature until transport to Zoolyx NV to evaluate the liver function.

Serum tubes (for antibody titer determination) were stored overnight at 2-8°C. Afterwards the supernatant was transferred to a new collection tube and centrifuged 10 minutes at 3400 g at 4°C. The top layer, being the serum, was transferred to a new tube, clearly labeled, and stored at -80°C. Any comments like serum volume and appearance of haemolysis were also noted. For this procedure IS-03-370 work instructions were followed.

### Liver function analysis

Whole blood was collected (D0 and D29) in the foreseen EDTA tubes (Zoolyx NV) and converted 3 times. The blood was transported to Zoolyx NV (Groeneweg 17, 9320 Erembodegem, Belgium) and analyzed the day of collection. Liver function analysis was performed by Zoolyx NV. This analysis studied AST, ALT, urea, creatinine, and triglyceride levels in the blood.

### RNA-Sequencing, Tissue processing, and analysis

After terminal bleeding, a small piece of the tumor was cut and isolated in a fresh sterile 1.5ml Eppendorf tube on ice. The tubes containing the tumor samples were stored at -80C freezer till shopping and further processing.

The Eppendorf tubes containing the samples were shipped to Diagnode (Belgium) on dry ice. The RNA-sequencing and analysis were performed by Diagnode.

### RNA-sequencing

To briefly describe the process, sequencing of the samples was performed on a Illumina NovaSeq 6000 instrument producing 50bp paired-end reads running Control Software 1.7.0. The sequenced reads were controlled for quality of sequencing with FastQC (Andrews, 2010. FastQC: A quality control tool for high throughput sequence data., 2010. URL : https://www.bioinformatics.babraham.ac.uk/projects/fastqc/) and trimmed using Cutadapt v3.5 (Martin et al., 2011. Cutadapt removes adapter sequences from high-throughput sequencing reads. EMBnet.journal, [S.I.], v. 17, n. 1, p. pp. 10-12, may 2011. ISSN 2226-6089.). Remaining reads reads were aligned to the reference genome mm10 using the STAR aligner version 2.7.9a (Dobin et al., 2013. STAR: ultrafast universal RNA-seq aligner. Bioinformatics (Oxford, England), 29(1):15-21, jan 2013. ISSN 1367-4811. doi: 10.1093/bioinformatics/bts635). RNA abundance was estimated with MGCount (Hita et al., 2022. MGcount: a total RNA-seq quantification tool to address multi-mapping and multi-overlapping alignments ambiguity in non-coding transcripts. BMC Bioinformatics, 23(1), January 2022. doi: 10.1186/s12859-021-04544-3.). This is a flexible quantification framework that firstly, it hierarchical assigns reads to transcripts to account for loci length disparity between small-RNA and long-RNA and secondly, it collapses loci where reads systematically multi-map into communities of loci.

### Statistical analysis:

Further analysis consider only features with counts per million (CPM) above 5 for at least 3 (total size of smallest groups) samples. Analysis performed included 1) Principal component analysis and clustering on the centered variance stabilization transformed count matrix. 2) Differential expression analysis with DESEQ2-pipeline (Love et al., 2014. Moderated estimation of fold change and dispersion for RNA-seq data with DESeq2. Genome Biology (2014) 15:550. DOI 10.1186/s13059-014-0550-8).

### Deviations

The protocol describes that during each feces collection 4 pellets would be collected (2 for processing, 2 for immediate -80°C storage). Collecting 4 pellets was not always possible.

The scoring sheet (with additional weight and food intake) is missing for D11.

BLI imaging was not performed on D21. To compensate, tumor volumes were assessed the day of sacrifice by caliper measurements ex vivo and photo documentation. The tumors were dissected and snap frozen on dry ice prior to transportation.

### Results and discussion

### 1. General observations

Test item preparation was performed according to the protocol. No deviations were observed during preparation. Test items had a normal, grainy and green appearance. Per dose of test item (3 gr), 0.6 gr nutella was used for mixing to assure proper dosing. Test items were dosed entirely overnight, no spillage in the cages of the mice was observed.

### 2. Body weight

Animals were weighted every day throughout the study. An overview of the evolution of mean weights per treatment group (WT and GMO) is shown on **Figure 3****.**

A drop in mean weight for both groups (WT and GMO) can be observed after each test item administration day. This was significant within the WT group when body weight of D8 versus D7 and D15 versus D14 were compared (P<0.05). The weight loss noted on these days did not lead to a humane endpoint. No statistical differences were found over time within the GMO group, nor between groups. The significant weight loss after compound dosing within the WT group could be explained by a decreased food intake and/or stress due to the single housing without regular food pellets overnight.

In addition, a slight increase in body weight over time is noted as a result of increased tumor growth and age (3). Mean weight of the animals increased throughout the study, from 18.16±0.91 gr to 20.0±1.67 gr for the WT group and from 18.76 ±1.21 gr to 20.95±2.4 gr for the GMO group.

### 3. Clinical observations

Clinical observations were all reported in the scoring sheets that were completed every day for the entire study duration. All aspects can be considered as normal for the mice. Several clinical observations were made during the study, including decreased grooming or ruffled fur, pale ears, a decreased mobility or depression/general weakness. These observations were mainly apparent towards the end of the study and mainly associated with the pathology. Overall, no humane endpoint nor significant changes in visual and behavioral parameters were observed the day after test items dosing apart from the significant weight loss detected in the WT group on D8 and D15.

### 4. Food intake

Pellets were weighted per cage every day throughout the study duration.

Food intake increased for all cages throughout the study. This increase could be explained by the applied pathology as tumor growth requires energy and an increased food intake. Despite the increased intake of food over time within the groups, it is noticeable that a decreased food intake is recorded the day after dosing in both study groups. This reduction in eating could be due to the compound dosing and the isolation overnight during the test item dosing, resulting in a potentially associated stress-induced change in caloric intake.

### 5. Tumor analysis

### 5.1. Presence of PiB001 in tumor samples

Dissected MC38 tumors from control and treated mice reveal presence of PiB001 in four out of five treated groups. Interestingly only mice with growing tumors showed the presence of PiB001 in them. PiB001 was detected using the 6xHis tag. Moreover, a band at 149kDa as well as multiple bands between 10-15 kDa were also observed (data not shown). These bands could imply protease cleavage of PiB001. However, enough of PiB001 reaches the site of MC-38 colon tumor draft to be detected and induce an immune response.

The results are shown on **Figure 4****.**

### 5.2. Tumor volume at D29

Twenty-nine days after randomization, mice were sacrificed and tumor lesions were assessed by means of caliper measurements. The average tumor volume is lower in the GMO treated group. The variation observed arises because a tumor did not grow in one mouse (WT group), while one tumor in GMO group grew rapidly (GMO group).

The results are shown on **Figure 5****.**

### 6. Analysis of blood ans fecal samples

### 6.1. Colon AST & ALT

The results of the liver and renal function analysis for WT group and GMO group are shown on **Figure 6****.**

AST and ALT levels remained within their physiological range at D0 (**Figure 6A**). On D29, the general AST value for mice of the WT group was above physiological levels (431 U/L compared to 397 U/L), whilst the ALT values remained normal. In addition, no abnormal changes in the fat metabolism was noted; the concentration of triglycerides in the blood remained within the physiological range (**Figure 6A**).

At D0 the levels of these enzymes are similar between the two groups. However at D29, AST and ALT serum levels in PiB001 treated group is lower than wild type plant treated group. This indicates that PiB001 group may have a protective effect compared to wild type plant treated mice because their colons are relatively healthier (**Figure 6A**)
Moreover, the De Ritis ratio between the two groups indicates that mice treated with PiB001 plant had 1.84x reduction in AST/ALT ratio between time points D0 and D29 when compared to mice treated with wild type plant. In addition these mice displayed a 22% decrease in AST/ALT ratio when compared to wild type plant fed mice (**Figure 6B**)

### 6.2. Glucose, Ureum & Creatinine serum levels

The results of Glucose, Ureum & Creatinine serum levels analysis for WT group and GMO group are shown on **Figure 7****.**

### Glucose concentrations

The results are shown on **Figure 7A****.**

Glucose concentrations were measured 1-2 hours after fasting the animals prior to blood collection and assessment. Glucose levels were measured before test item dosing on D0 and after the last dosing on D29 (end of the study). Glucose levels ranged from 150.8 mg/dl for mice of the WT group to 132.8 mg/dl for mice of the GMO group on average on D0. Small and non-significant changes in these glucose concentrations occurred over time, with a mean glucose level of 148.8 mg/dl for mice of the WT group and 129 mg/dl glucose in mice of the GMO group. In addition, no statistical differences were present between groups.

### Creatinine and ureum values

The results are shown on **Figure 7B** and **Figure 7C****.**

Creatinine and Ureum values remained within the range of reference values/physiological range as well (**Figure 7B and Figure 7C**). The physiological value of AST on D29 could be the result of several parameters including haemolysis of the blood, lipemia or liver damage. Because the values for ALT and triglycerides remained within the normal range, blood haemolysis would explain the elevated AST concentration.

### Sum up

To sum up, blood analysis of the mice enrolled in the study show overall normal metabolic parameters (e.g. glucose, ALT and triglyceride levels etc.) indicating that dosing of plant material (both WT and GMO) on a weekly basis was well tolerated. In addition, analysis of the fecal matter indicated no changes in antibody titers, potentially indicating no significant alterations in intestinal flora.

### 6.3. Immunoassay antibody (IgA, IgM) analysis of the blood samples

Serum IgA & IgM levels were assayed at D0 and D29.

The results are shown on **Figure 8****.**

The graphs show IgA & IgM titers in the serum. At day 29, PiB001 treated mice group displayed twice the serum titer compared to WT plant treated group. This indicates that PiB001 was able to stimulate intestinal immune system to secrete IgA. This IgA was of serum type which is detectable in the blood. However, no significant increase was observed in the serum IgM levels.

### 6.4. Immunoassay antibody (IgA, IgM) analysis of the feces samples

Feces IgA & IgM levels were assayed from D0 to D29.

The results are shown on **Figure 9****.**

No significant differences were observed between the groups for the different tested antibody titers, including for IgA and IgM. No statistical analysis on serum samples collected on D0 (prior to dosing) and D29 could be performed because of the use of pooled samples. Two different dilutions were applied when performing the immunoassay.

The graphs display fecal titers of IgM and IgA antibodies. (**Figure 9A**) No significant increase was observed in fecal IgA levels between the two groups over the course of the treatment. (Figu**r**e **9B**) The graph shows the measured response of IgMs for the duration of the experiment. The IgM titers continued to increase from day 8 onwards. For the course of the experiment IgM level continued to rise with continued treatments. At day 22, IgM titer was 6.5 times higher than the control. A steady rise in IgM titer indicates that PiB001 antibody induced an IgM response from interstitial immune cells.

### 7. Immunochemistry (IHC) assays

### 7.1. IHC of Normal Human Colon revealing CD4+ cells in the Colon.

Sections of health human colons were purchased (Amsbio T1234090) and stored at -80C. IHC was preformed on these sections as following. The samples were brought to room temperature and OTC was washed with 1XPBS. The sections were permeabilized with 1XPBS containing 0.1% tritonX-100 for 30 minutes. The sections were washed with 1XPBS three times for 5 minutes each time. Then antigen retrieval step was preformed in 1X PBS and 100mM glycine for 30 minutes. The sections were washed again with 1X PBS three times for 10 minutes each time. Following the blocking step with a buffer containing 1XPBS, 10%FBS, and 0.5% Triton at room temperture and pressure for 1 hour, primary antibodies were added to the sections. Primary antibodies (Bioledgend mouse anti human CD4) was diluted 1:500 in a solution containing 1XPBS, 10%FBS, and 0.5 % tritonX-100. The primary antibody probing was preformed overnight at 4C in a humid chamber. The next day the primary antibodies were washed with 1XPBS 3 times for 5 minutes each. The washing step was followed by secondary antibody labeling (Thermofischer goat anti-mouse 647 1:1000) prepared in the same buffer as the blocking buffer for 1 hour. After 1 hour of incubation at room temperture the sections were washing with 3 times with 1XPBS for 5 minutes each. Then the sections were mounted with mounting media containing DAPI (Thermofischer 00-4959-52) and let to dry in a dark chamber.

The results are shown on **Figure 10****.**

This study was preformed to demonstrate that in human colon, immune cells such as CD4+ cells were present near the intestinal epithelium and that it might be possible to activate them using orally delivered immunotherapeutic agents such as nivolumab. In addition, gut-associated lymphoid tissue is also present near the lumen of the intestine. This tissue is rich is various type of white bloods cells. These can also be targeted to activate the immune cells.

### 7.2. IHC on human Colon with adenocarcinoma comparing binding of Nivolumab and PiB001.

Sections of diseased human colons containing adenocarcinoma were purchased (Amsbio T1235090) and stored at -80°C. IHC was preformed on these sections as following. The samples were brought to room temperature and OTC was washed with 1XPBS. The sections were permeabilized with 1XPBS containing 0.1% tritonX-100 for 30 minutes. The sections were washed with 1XPBS three times for 5 minutes each time. Then antigen retrieval step was preformed in 1X PBS and 100mM glycine for 30 minutes. The sections were washed again with 1X PBS three times for 10 minutes each time. Following the blocking step with a buffer containing 1XPBS, 10%FBS, and 0.5% Triton at room temperature and pressure for 1 hour. Fresh cabbage leaves were used to extract PiB001 (primary antibody) from the leaves. The crude plant extract was filtered through a 0.2um filter and the product was diluted 1/100 (in fresh blocking buffer). The sections were incubated overnight with primary antibodies (PiB001 or nivolumab) at 4C in a humid chamber. Sections were stained with nivolumab (Thermofischer MA541974 1:100) as a control. The next day the primary antibodies were washed with 1XPBS 3 times for 5 minutes each. The washing step was followed by applying secondary antibodies (Thermofischer mouse anti Nivolumab 1/500 & Goat anti mouse 555 1/1000) for 1 hours at RTP. After 1 hour of incubation at room temperature the sections were washing 3 times with 1XPBS for 5 minutes each. Then the sections were mounted with mounting media containing DAPI (Thermofischer 00-4959-52) and let to dry in a dark chamber.

The results are shown on **Figure 11****.**

The purpose of this experiment was to demonstrate that PiB001 was able to recognize PD-1+ immune cells in diseased human colon (adenocarcinoma), in a similar manner as mammalian cell produced nivolumab (anti-PD1). The results show that crude PiB001 was able to recognize PD-1+ cells in the gut-associated lymphoid tissue but also tumor infiltrated immune cells.

### 8. RNA analysis

### 8.1. PCA based on RNA-seq of dissected MC-38 tumors.

Raw counts were transformed following the variance stabilization approach, centered and scaled. A principal component analysis was performed to explore the patterns in a reduced dimensionality space.

The results are shown on **Figure 12****.**

**Figure 12** shows the data points in the sub-space defined by the two first principal components. RNAseq and bioinformatic services were performed by Diagnode Belgium.

### 8.2. Differential gene expression between tumors treated with WT plants and PiB001.

The WT vs GMO comparison showed 7 differentially expressed features with a minimum log2 fold-change of 1 at a significance level of P-adj < 0.01. The Volcano plot in fig. 5 presents the log2 fold-change in the x-axis while the y-axis represents the significance of the differential expression for each transcriptomic feature.

The results are shown on **Figure 13****.**

The Heatmap plot of Figure 13 shows the 50 top features manifesting the highest differential effect in each direction. RNAseq and bioinformatic services were preformed by Diagnode Belgium.

### Conclusion

Overall, the test items were well tolerated by the mice when a weekly dosing schedule was applied. Test item dosing overall did not impact the metabolic parameters (e.g. creatinine, ureum, tryglicerides) nor the glucose level in the blood. These results demonstrate that the product did not impact the liver, kidneys, and the pancreas.

Moreover, clinical symptoms occurring during the study were mainly associated with the disease pathology as they were observed randomly and in a more frequent manner over time and not specifically the day after dosing. Weight loss was an exception, with mice of the WT group significantly losing weight on D8 and D15. The mice showed an increased tumor growth over time based on both BLI imaging and caliper measurements ex vivo. However, test item administration slowed down the tumor growth but did not eradicate it.

The GMO treated mice demonstrated increased levels of fecal IgM and serum IgA. Increased levels of these antibodies suggest a response by intestinal immune cells. IgM antibodies can be a response from intestinal B lymphocytes, while serum IgA reflects on activity by the intestinal plasma cells. Moreover the B cell response could have originated from the lamina propria or cells resident in the intestinal lymph nodes.

**Table 4: SEQUENCES**

| | |
|---|---|
| SEQ ID NO:1 (Nivolumab VH sequence) | QVQLVESGGGVVQPGRSLRLDCKASGITFSNSGMHWVRQAPGKGLE WVAVIWYDGSKRYYADSVKGRFTISRDNSKNTLFLQMNSLRAEDTAVY YCATNDDYWGQGTLVTVSSASTKGPSVFPLAPCSRSTSESTAALGCLV KDYFPEPVTVSWNSGALTSGVHTFPAVLQSSGLYSLSSVVTVPSSSLG TKTYTCNVDHKPSNTKVDKRVESKYGPPCPPCPAPEFLGGPSVFLFPP KPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPE NNYKTTPPVLDSDGSFFLYSRLTVDKSRWQEGNVFSCSVMHEALHNH YTQKSLSLSLGK |
| SEQ ID NO:2 (Nivolumab VL sequence) | EIVLTQSPATLSLSPGERATLSCRASQSVSSYLAWYQQKPGQAPRLLIY DASNRATGIPARFSGSGSGTDFTLTISSLEPEDFAVYYCQQSSNWPRT FGQGTKVEIKRTVAAPSVFIFPPSDEQLKSGTASVVCLLNNFYPREAKV QWKVDNALQSGNSQESVTEQDSKDSTYSLSSTLTLSKADYEKHKVYA CEVTHQGLSSPVTKSFN RGEC |
| SEQ ID NO:3 Nivolumab construct sequence | EIVLTQSPATLSLSPGERATLSCRASQSVSSYLAWYQQKPGQAPRLLIY DASNRATGIPARFSGSGSGTDFTLTISSLEPEDFAVYYCQQSSNWPRT FGQGTKVEIKRTVAAPSVFIFPPSDEQLKSGTASVVCLLNNFYPREAKV QWKVDNALQSGNSQESVTEQDSKDSTYSLSSTLTLSKADYEKHKVYA CEVTHQGLSSPVTKSFNRGECHHHHHHQVQLVESGGGVVQPGRSLR LDCKASGITFSNSGMHWVRQAPGKGLEWVAVIWYDGSKRYYADSVKG RFTISRDNSKNTLFLQMNSLRAEDTAVYYCATNDDYWGQGTLVTVSSA STKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKR VESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQD WLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYS RLTVDKSRWQEGNVFSCSVMHEALHNHYTQKSLSLSLGKYPYDVPDY AYPYDVPDYA |
| SEQ ID NO:4 Optimized coding sequence for Nivolumab (minusTags) | ATGGAAATCGTTCTTACACAGTCACCTGCTACTCTTTCTCTTTCACCA GGTGAGAGAGCTACTCTTTCATGTAGAGCTAGCCAATCTGTTTCTTC TTATTTGGCTTGGTATCAGCAAAAACCTGGTCAAGCTCCTAGATTGC TTATCTACGATGCAAGTAACCGGGCTACAGGAATTCCTGCTAGATTT TCAGGATCTGGATCCGGTACTGATTTCACTCTCACAATTTCCTCTTT GGAACCTGAAGATTTCGCTGTTTATTACTGTCAACAATCTAGTAATT GGCCAAGAACTTTTGGACAGGGAACTAAGGTTGAGATTAAAAGGAC TGTTGCTGCTCCTAGTGTGTTTATCTTTCCTCCATCTGATGAACAATT |
| | GAAGTCTGGTACAGCTTCAGTGGTTTGTCTTCTTAACAATTTTTACC CTAGAGAGGCTAAAGTTCAGTGGAAAGTGGATAACGCTTTGCAATC TGGTAACTCTCAAGAATCTGTTACTGAGCAAGATTCCAAAGATAGTA CCTACTCTCTTAGTTCAACACTTACATTGTCCAAGGCAGATTATGAG AAACATAAAGTGTATGCTTGCGAGGTGACTCATCAGGGACTCTCTTC TCCTGTTACTAAGTCTTTTAACAGAGGAGAGTGTCATCACCATCATC ATCATCAAGTTCAGTTGGTGGAAAGCGGAGGAGGAGTTGTTCAGCC TGGTAGGTCTCTCAGACTTGATTGCAAGGCTTCTGGTATTACTTTTA GTAACTCTGGAATGCATTGGGTTAGACAAGCTCCTGGAAAAGGTCT TGAATGGGTTGCTGTTATTTGGTATGATGGATCTAAGAGATACTACG CTGATTCAGTTAAGGGAAGATTCACAATTTCTAGAGATAACTCTAAA AATACTCTTTTCCTTCAAATGAATTCATTAAGAGCAGAGGATACTGCA GTGTATTACTGCGCTACCAATGATGATTATTGGGGTCAGGGAACACT TGTTACTGTTTCTTCTGCTTCTACTAAAGGACCATCTGTTTTTCCACT GGCTCCTTGTTCGAGATCTACATCTGAATCAACTGCAGCTCTTGGAT GTCTTGTTAAAGATTATTTCCCAGAGCCTGTTACTGTTTCTTGGAATT CTGGTGCTCTTACATCTGGTGTTCATACGTTCCCTGCTGTTCTTCAA TCTTCTGGATTGTACTCACTCTCATCTGTTGTTACAGTTCCTTCATCT AGTCTTGGTACTAAAACATATACATGTAATGTTGATCATAAGCCTAGT AATACTAAGGTTGATAAAAGAGTTGAGTCCAAGTATGGTCCACCTTG TCCACCTTGTCCTGCTCCGGAGTTCCTTGGTGGACCTTCTGTTTTTC TTTTCCCTCCAAAGCCTAAGGACACTCTTATGATTTCGAGAACTCCT GAGGTTACTTGTGTTGTGGTGGATGTGTCACAAGAGGATCCTGAAG TTCAGTTTAACTGGTATGTTGATGGAGTTGAGGTTCATAATGCTAAG ACTAAGCCTAGAGAAGAACAATTCAACTCTACTTACAGAGTTGTTTC AGTGTTAACTGTTCTTCATCAGGATTGGCTTAATGGTAAGGAGTATA AGTGCAAGGTTTCTAACAAGGGACTTCCATCATCAATAGAGAAGACT ATTTCTAAGGCTAAGGGTCAGCCAAGAGAACCTCAGGTTTACACTCT TCCACCTTCTCAGGAAGAAATGACTAAGAACCAAGTTTCATTAACAT GTTTAGTCAAAGGTTTCTACCCTTCAGATATTGCTGTTGAGTGGGAA TCTAATGGACAGCCTGAGAACAACTATAAGACTACACCACCTGTTTT GGATTCTGATGGATCATTTTTCCTTTACTCTAGATTGACAGTGGATAA ATCTAGGTGGCAAGAAGGAAATGTTTTTTCTTGTAGTGTGATGCATG AGGCGTTGCATAACCATTACACACAAAAAAGTTTGTCTCTTTCACTT GGTAAGTATCCATACGATGTTCCTGACTATGCTTATCCTTATGATGTT CCAGATTATGCTTGA ATGGAAATTGTGCTGACCCAGAGCCCGGCGACCCTGAGCCTGAGC CCGGGCGAACGCGCGACCCTGAGCTGCCGCGCGAGCCAGAGCGT GAGCAGCTATCTGGCGTGGTATCAGCAGAAACCGGGCCAGGCGCC GCGCCTGCTGATTTATGATGCGAGCAACCGCGCGACCGGCATTCC GGCGCGCTTTAGCGGCAGCGGCAGCGGCACCGATTTTACCCTGAC CATTAGCAGCCTGGAACCGGAAGATTTTGCGGTGTATTATTGCCAG CAGAGCAGCAACTGGCCGCGCACCTTTGGCCAGGGCACCAAAGTG GAAATTAAACGCACCGTGGCGGCGCCGAGCGTGTTTATTTTTCCGC CGAGCGATGAACAGCTGAAAAGCGGCACCGCGAGCGTGGTGTGCC TGCTGAACAACTTTTATCCGCGCGAAGCGAAAGTGCAGTGGAAAGT GGATAACGCGCTGCAGAGCGGCAACAGCCAGGAAAGCGTGACCGA ACAGGATAGCAAAGATAGCACCTATAGCCTGAGCAGCACCCTGACC CTGAGCAAAGCGGATTATGAAAAACATAAAGTGTATGCGTGCGAAG TGACCCATCAGGGCCTGAGCAGCCCGGTGACCAAAAGCTTTAACCG CGGCGAATGCCAGGTGCAGCTGGTGGAAAGCGGCGGCGGCGTGG TGCAGCCGGGCCGCAGCCTGCGCCTGGATTGCAAAGCGAGCGGCA TTACCTTTAGCAACAGCGGCATGCATTGGGTGCGCCAGGCGCCGG GCAAAGGCCTGGAATGGGTGGCGGTGATTTGGTATGATGGCAGCA AACGCTATTATGCGGATAGCGTGAAAGGCCGCTTTACCATTAGCCG |
| SEQ ID NO:5 Non-optimized Nivolumab (minus Tags) coding sequence (nt) | ATGGAAATTGTGCTGACCCAGAGCCCGGCGACCCTGAGCCTGAGC CCGGGCGAACGCGCGACCCTGAGCTGCCGCGCGAGCCAGAGCGT GAGCAGCTATCTGGCGTGGTATCAGCAGAAACCGGGCCAGGCGCC GCGCCTGCTGATTTATGATGCGAGCAACCGCGCGACCGGCATTCC GGCGCGCTTTAGCGGCAGCGGCAGCGGCACCGATTTTACCCTGAC CATTAGCAGCCTGGAACCGGAAGATTTTGCGGTGTATTATTGCCAG CAGAGCAGCAACTGGCCGCGCACCTTTGGCCAGGGCACCAAAGTG GAAATTAAACGCACCGTGGCGGCGCCGAGCGTGTTTATTTTTCCGC CGAGCGATGAACAGCTGAAAAGCGGCACCGCGAGCGTGGTGTGCC TGCTGAACAACTTTTATCCGCGCGAAGCGAAAGTGCAGTGGAAAGT GGATAACGCGCTGCAGAGCGGCAACAGCCAGGAAAGCGTGACCGA ACAGGATAGCAAAGATAGCACCTATAGCCTGAGCAGCACCCTGACC CTGAGCAAAGCGGATTATGAAAAACATAAAGTGTATGCGTGCGAAG TGACCCATCAGGGCCTGAGCAGCCCGGTGACCAAAAGCTTTAACCG CGGCGAATGCCAGGTGCAGCTGGTGGAAAGCGGCGGCGGCGTGG TGCAGCCGGGCCGCAGCCTGCGCCTGGATTGCAAAGCGAGCGGCA TTACCTTTAGCAACAGCGGCATGCATTGGGTGCGCCAGGCGCCGG GCAAAGGCCTGGAATGGGTGGCGGTGATTTGGTATGATGGCAGCA AACGCTATTATGCGGATAGCGTGAAAGGCCGCTTTACCATTAGCCG |
| | CGATAACAGCAAAAACACCCTGTTTCTGCAGATGAACAGCCTGCGC GCGGAAGATACCGCGGTGTATTATTGCGCGACCAACGATGATTATT GGGGCCAGGGCACCCTGGTGACCGTGAGCAGCGCGAGCACCAAA GGCCCGAGCGTGTTTCCGCTGGCGCCGTGCAGCCGCAGCACCAGC GAAAGCACCGCGGCGCTGGGCTGCCTGGTGAAAGATTATTTTCCG GAACCGGTGACCGTGAGCTGGAACAGCGGCGCGCTGACCAGCGG CGTGCATACCTTTCCGGCGGTGCTGCAGAGCAGCGGCCTGTATAG CCTGAGCAGCGTGGTGACCGTGCCGAGCAGCAGCCTGGGCACCAA AACCTATACCTGCAACGTGGATCATAAACCGAGCAACACCAAAGTG GATAAACGCGTGGAAAGCAAATATGGCCCGCCGTGCCCGCCGTGC CCGGCGCCGGAATTTCTGGGCGGCCCGAGCGTGTTTCTGTTTCCG CCGAAACCGAAAGATACCCTGATGATTAGCCGCACCCCGGAAGTGA CCTGCGTGGTGGTGGATGTGAGCCAGGAAGATCCGGAAGTGCAGT TTAACTGGTATGTGGATGGCGTGGAAGTGCATAACGCGAAAACCAA ACCGCGCGAAGAACAGTTTAACAGCACCTATCGCGTGGTGAGCGTG CTGACCGTGCTGCATCAGGATTGGCTGAACGGCAAAGAATATAAAT GCAAAGTGAGCAACAAAGGCCTGCCGAGCAGCATTGAAAAAACCAT TAGCAAAGCGAAAGGCCAGCCGCGCGAACCGCAGGTGTATACCCT GCCGCCGAGCCAGGAAGAAATGACCAAAAACCAGGTGAGCCTGAC CTGCCTGGTGAAAGGCTTTTATCCGAGCGATATTGCGGTGGAATGG GAAAGCAACGGCCAGCCGGAAAACAACTATAAAACCACCCCGCCG GTGCTGGATAGCGATGGCAGCTTTTTTCTGTATAGCCGCCTGACCG TGGATAAAAGCCGCTGGCAGGAAGGCAACGTGTTTAGCTGCAGCGT GATGCATGAAGCGCTGCATAACCATTATACCCAGAAAAGCCTGAGC CTGAGCCTGGGCAAA |
| SEQ ID NO:6 Nucleic acid construct CaMV35S-Nivolumab (Optimized for Arabidopsis Thaliana but works in cabbage) +promoter, +tags | GCCAACATGGTGGAGCACGACACTCTCGTCTACTCCAAGAATATCA AAGATACAGTCTCAGAAGACCAAAGGGCTATTGAGACTTTTCAACAA AGGGTAATATCGGGAAACCTCCTCGGATTCCATTGCCCAGCTATCT GTCACTTCATCAAAAGGACAGTAGAAAAGGAAGGTGGCACCTACAA ATGCCATCATTGCGATAAAGGAAAGGCTATCGTTCAAGATGCCTCTG CCGACAGTGGTCCCAAAGATGGACCCCCACCCACGAGGAGCATCG TGGAAAAAGAAGACGTTCCAACCACGTCTTCAAAGCAAGTGGATTG ATGTGAACATGGTGGAGCACGACACTCTCGTCTACTCCAAGAATAT CAAAGATACAGTCTCAGAAGACCAAAGGGCTATTGAGACTTTTCAAC AAAGGGTAATATCGGGAAACCTCCTCGGATTCCATTGCCCAGCTAT CTGTCACTTCATCAAAAGGACAGTAGAAAAGGAAGGTGGCACCTAC AAATGCCATCATTGCGATAAAGGAAAGGCTATCGTTCAAGATGCCTC TGCCGACAGTGGTCCCAAAGATGGACCCCCACCCACGAGGAGCAT CGTGGAAAAAGAAGACGTTCCAACCACGTCTTCAAAGCAAGTGGAT TGATGTGATATCTCCACTGACGTAAGGGATGACGCACAATCCCACT ATCCTTCGCAAGACCCTTCCTCTATATAAGGAAGTTCATTTCATTTG GAGAGGACACGCTGAATGGAAATCGTTCTTACACAGTCACCTGCTA CTCTTTCTCTTTCACCAGGTGAGAGAGCTACTCTTTCATGTAGAGCT AGCCAATCTGTTTCTTCTTATTTGGCTTGGTATCAGCAAAAACCTGG TCAAGCTCCTAGATTGCTTATCTACGATGCAAGTAACCGGGCTACAG GAATTCCTGCTAGATTTTCAGGATCTGGATCCGGTACTGATTTCACT CTCACAATTTCCTCTTTGGAACCTGAAGATTTCGCTGTTTATTACTGT CAACAATCTAGTAATTGGCCAAGAACTTTTGGACAGGGAACTAAGGT TGAGATTAAAAGGACTGTTGCTGCTCCTAGTGTGTTTATCTTTCCTC CATCTGATGAACAATTGAAGTCTGGTACAGCTTCAGTGGTTTGTCTT CTTAACAATTTTTACCCTAGAGAGGCTAAAGTTCAGTGGAAAGTGGA TAACGCTTTGCAATCTGGTAACTCTCAAGAATCTGTTACTGAGCAAG ATTCCAAAGATAGTACCTACTCTCTTAGTTCAACACTTACATTGTCCA AGGCAGATTATGAGAAACATAAAGTGTATGCTTGCGAGGTGACTCAT CAGGGACTCTCTTCTCCTGTTACTAAGTCTTTTAACAGAGGAGAGTG |
| | TCATCACCATCATCATCATCAAGTTCAGTTGGTGGAAAGCGGAGGA GGAGTTGTTCAGCCTGGTAGGTCTCTCAGACTTGATTGCAAGGCTT CTGGTATTACTTTTAGTAACTCTGGAATGCATTGGGTTAGACAAGCT CCTGGAAAAGGTCTTGAATGGGTTGCTGTTATTTGGTATGATGGATC TAAGAGATACTACGCTGATTCAGTTAAGGGAAGATTCACAATTTCTA GAGATAACTCTAAAAATACTCTTTTCCTTCAAATGAATTCATTAAGAG CAGAGGATACTGCAGTGTATTACTGCGCTACCAATGATGATTATTGG GGTCAGGGAACACTTGTTACTGTTTCTTCTGCTTCTACTAAAGGACC ATCTGTTTTTCCACTGGCTCCTTGTTCGAGATCTACATCTGAATCAA CTGCAGCTCTTGGATGTCTTGTTAAAGATTATTTCCCAGAGCCTGTT ACTGTTTCTTGGAATTCTGGTGCTCTTACATCTGGTGTTCATACGTT CCCTGCTGTTCTTCAATCTTCTGGATTGTACTCACTCTCATCTGTTGT TACAGTTCCTTCATCTAGTCTTGGTACTAAAACATATACATGTAATGT TGATCATAAGCCTAGTAATACTAAGGTTGATAAAAGAGTTGAGTCCA AGTATGGTCCACCTTGTCCACCTTGTCCTGCTCCGGAGTTCCTTGG TGGACCTTCTGTTTTTCTTTTCCCTCCAAAGCCTAAGGACACTCTTAT GATTTCGAGAACTCCTGAGGTTACTTGTGTTGTGGTGGATGTGTCAC AAGAGGATCCTGAAGTTCAGTTTAACTGGTATGTTGATGGAGTTGAG GTTCATAATGCTAAGACTAAGCCTAGAGAAGAACAATTCAACTCTAC TTACAGAGTTGTTTCAGTGTTAACTGTTCTTCATCAGGATTGGCTTAA TGGTAAGGAGTATAAGTGCAAGGTTTCTAACAAGGGACTTCCATCAT CAATAGAGAAGACTATTTCTAAGGCTAAGGGTCAGCCAAGAGAACC TCAGGTTTACACTCTTCCACCTTCTCAGGAAGAAATGACTAAGAACC AAGTTTCATTAACATGTTTAGTCAAAGGTTTCTACCCTTCAGATATTG CTGTTGAGTGGGAATCTAATGGACAGCCTGAGAACAACTATAAGAC TACACCACCTGTTTTGGATTCTGATGGATCATTTTTCCTTTACTCTAG ATTGACAGTGGATAAATCTAGGTGGCAAGAAGGAAATGTTTTTTCTT GTAGTGTGATGCATGAGGCGTTGCATAACCATTACACACAAAAAAGT TTGTCTCTTTCACTTGGTAAGTATCCATACGATGTTCCTGACTATGCT TATCCTTATGATGTTCCAGATTATGCTTGA |
| SEQ ID NO:7 Nucleic acid construct CaMV35S-Nivolumab (original) (with promoter tags) | GCCAACATGGTGGAGCACGACACTCTCGTCTACTCCAAGAATATCA AAGATACAGTCTCAGAAGACCAAAGGGCTATTGAGACTTTTCAACAA AGGGTAATATCGGGAAACCTCCTCGGATTCCATTGCCCAGCTATCT GTCACTTCATCAAAAGGACAGTAGAAAAGGAAGGTGGCACCTACAA ATGCCATCATTGCGATAAAGGAAAGGCTATCGTTCAAGATGCCTCTG CCGACAGTGGTCCCAAAGATGGACCCCCACCCACGAGGAGCATCG TGGAAAAAGAAGACGTTCCAACCACGTCTTCAAAGCAAGTGGATTG ATGTGAACATGGTGGAGCACGACACTCTCGTCTACTCCAAGAATAT CAAAGATACAGTCTCAGAAGACCAAAGGGCTATTGAGACTTTTCAAC AAAGGGTAATATCGGGAAACCTCCTCGGATTCCATTGCCCAGCTAT CTGTCACTTCATCAAAAGGACAGTAGAAAAGGAAGGTGGCACCTAC AAATGCCATCATTGCGATAAAGGAAAGGCTATCGTTCAAGATGCCTC TGCCGACAGTGGTCCCAAAGATGGACCCCCACCCACGAGGAGCAT CGTGGAAAAAGAAGACGTTCCAACCACGTCTTCAAAGCAAGTGGAT TGATGTGATATCTCCACTGACGTAAGGGATGACGCACAATCCCACT ATCCTTCGCAAGACCCTTCCTCTATATAAGGAAGTTCATTTCATTTG GAGAGGACACGCTGAATGGAAATTGTGCTGACCCAGAGCCCGGCG ACCCTGAGCCTGAGCCCGGGCGAACGCGCGACCCTGAGCTGCCG CGCGAGCCAGAGCGTGAGCAGCTATCTGGCGTGGTATCAGCAGAA ACCGGGCCAGGCGCCGCGCCTGCTGATTTATGATGCGAGCAACCG CGCGACCGGCATTCCGGCGCGCTTTAGCGGCAGCGGCAGCGGCA CCGATTTTACCCTGACCATTAGCAGCCTGGAACCGGAAGATTTTGC GGTGTATTATTGCCAGCAGAGCAGCAACTGGCCGCGCACCTTTGGC CAGGGCACCAAAGTGGAAATTAAACGCACCGTGGCGGCGCCGAGC GTGTTTATTTTTCCGCCGAGCGATGAACAGCTGAAAAGCGGCACCG |
| | CGAGCGTGGTGTGCCTGCTGAACAACTTTTATCCGCGCGAAGCGAA AGTGCAGTGGAAAGTGGATAACGCGCTGCAGAGCGGCAACAGCCA GGAAAGCGTGACCGAACAGGATAGCAAAGATAGCACCTATAGCCTG AGCAGCACCCTGACCCTGAGCAAAGCGGATTATGAAAAACATAAAG TGTATGCGTGCGAAGTGACCCATCAGGGCCTGAGCAGCCCGGTGA CCAAAAGCTTTAACCGCGGCGAATGCCACCACCACCACCACCACCA GGTGCAGCTGGTGGAAAGCGGCGGCGGCGTGGTGCAGCCGGGCC GCAGCCTGCGCCTGGATTGCAAAGCGAGCGGCATTACCTTTAGCAA CAGCGGCATGCATTGGGTGCGCCAGGCGCCGGGCAAAGGCCTGG AATGGGTGGCGGTGATTTGGTATGATGGCAGCAAACGCTATTATGC GGATAGCGTGAAAGGCCGCTTTACCATTAGCCGCGATAACAGCAAA AACACCCTGTTTCTGCAGATGAACAGCCTGCGCGCGGAAGATACCG CGGTGTATTATTGCGCGACCAACGATGATTATTGGGGCCAGGGCAC CCTGGTGACCGTGAGCAGCGCGAGCACCAAAGGCCCGAGCGTGTT TCCGCTGGCGCCGTGCAGCCGCAGCACCAGCGAAAGCACCGCGG CGCTGGGCTGCCTGGTGAAAGATTATTTTCCGGAACCGGTGACCGT GAGCTGGAACAGCGGCGCGCTGACCAGCGGCGTGCATACCTTTCC GGCGGTGCTGCAGAGCAGCGGCCTGTATAGCCTGAGCAGCGTGGT GACCGTGCCGAGCAGCAGCCTGGGCACCAAAACCTATACCTGCAA CGTGGATCATAAACCGAGCAACACCAAAGTGGATAAACGCGTGGAA AGCAAATATGGCCCGCCGTGCCCGCCGTGCCCGGCGCCGGAATTT CTGGGCGGCCCGAGCGTGTTTCTGTTTCCGCCGAAACCGAAAGATA CCCTGATGATTAGCCGCACCCCGGAAGTGACCTGCGTGGTGGTGG ATGTGAGCCAGGAAGATCCGGAAGTGCAGTTTAACTGGTATGTGGA TGGCGTGGAAGTGCATAACGCGAAAACCAAACCGCGCGAAGAACA GTTTAACAGCACCTATCGCGTGGTGAGCGTGCTGACCGTGCTGCAT CAGGATTGGCTGAACGGCAAAGAATATAAATGCAAAGTGAGCAACA AAGGCCTGCCGAGCAGCATTGAAAAAACCATTAGCAAAGCGAAAGG CCAGCCGCGCGAACCGCAGGTGTATACCCTGCCGCCGAGCCAGGA AGAAATGACCAAAAACCAGGTGAGCCTGACCTGCCTGGTGAAAGGC TTTTATCCGAGCGATATTGCGGTGGAATGGGAAAGCAACGGCCAGC CGGAAAACAACTATAAAACCACCCCGCCGGTGCTGGATAGCGATGG CAGCTTTTTTCTGTATAGCCGCCTGACCGTGGATAAAAGCCGCTGG CAGGAAGGCAACGTGTTTAGCTGCAGCGTGATGCATGAAGCGCTG CATAACCATTATACCCAGAAAAGCCTGAGCCTGAGCCTGGGCAAAT ACCCATACGATGTTCCAGATTACGCTTATCCATATGATGTTCCAGAT TATGCTTAA GATCATGAGCGGAGAATTAAGGGAGTCACGTTATGACCCCCGCCGA TGACGCGGGACAAGCCGTTTTACGTTTGGAACTGACAGAACCGCAA CGTTGAAGGAGCCACTCAGCCGCGGGTTTCTGGAGTTTAATGAGCT AAGCACATACGTCAGAAACCATTATTGCGCGTTCAAAAGTCGCCTAA GGTCACTATCAGCTAGCAAATATTTCTTGTCAAAAATGCTCCACTGA CGTTCCATAAATTCCCCTCGGTATCCAATTAGAGTCTCATATTCACT CTCAATCCAAATAATCTGCAATGGAAATTGTGCTGACCCAGAGCCC GGCGACCCTGAGCCTGAGCCCGGGCGAACGCGCGACCCTGAGCT GCCGCGCGAGCCAGAGCGTGAGCAGCTATCTGGCGTGGTATCAGC AGAAACCGGGCCAGGCGCCGCGCCTGCTGATTTATGATGCGAGCA ACCGCGCGACCGGCATTCCGGCGCGCTTTAGCGGCAGCGGCAGC GGCACCGATTTTACCCTGACCATTAGCAGCCTGGAACCGGAAGATT TTGCGGTGTATTATTGCCAGCAGAGCAGCAACTGGCCGCGCACCTT TGGCCAGGGCACCAAAGTGGAAATTAAACGCACCGTGGCGGCGCC GAGCGTGTTTATTTTTCCGCCGAGCGATGAACAGCTGAAAAGCGGC ACCGCGAGCGTGGTGTGCCTGCTGAACAACTTTTATCCGCGCGAAG CGAAAGTGCAGTGGAAAGTGGATAACGCGCTGCAGAGCGGCAACA GCCAGGAAAGCGTGACCGAACAGGATAGCAAAGATAGCACCTATAG |
| SEQ ID NO:8 Nucleic acid construct (Nos-nivolumab) with promoter and tags | GATCATGAGCGGAGAATTAAGGGAGTCACGTTATGACCCCCGCCGA TGACGCGGGACAAGCCGTTTTACGTTTGGAACTGACAGAACCGCAA CGTTGAAGGAGCCACTCAGCCGCGGGTTTCTGGAGTTTAATGAGCT AAGCACATACGTCAGAAACCATTATTGCGCGTTCAAAAGTCGCCTAA GGTCACTATCAGCTAGCAAATATTTCTTGTCAAAAATGCTCCACTGA CGTTCCATAAATTCCCCTCGGTATCCAATTAGAGTCTCATATTCACT CTCAATCCAAATAATCTGCAATGGAAATTGTGCTGACCCAGAGCCC GGCGACCCTGAGCCTGAGCCCGGGCGAACGCGCGACCCTGAGCT GCCGCGCGAGCCAGAGCGTGAGCAGCTATCTGGCGTGGTATCAGC AGAAACCGGGCCAGGCGCCGCGCCTGCTGATTTATGATGCGAGCA ACCGCGCGACCGGCATTCCGGCGCGCTTTAGCGGCAGCGGCAGC GGCACCGATTTTACCCTGACCATTAGCAGCCTGGAACCGGAAGATT TTGCGGTGTATTATTGCCAGCAGAGCAGCAACTGGCCGCGCACCTT TGGCCAGGGCACCAAAGTGGAAATTAAACGCACCGTGGCGGCGCC GAGCGTGTTTATTTTTCCGCCGAGCGATGAACAGCTGAAAAGCGGC ACCGCGAGCGTGGTGTGCCTGCTGAACAACTTTTATCCGCGCGAAG CGAAAGTGCAGTGGAAAGTGGATAACGCGCTGCAGAGCGGCAACA GCCAGGAAAGCGTGACCGAACAGGATAGCAAAGATAGCACCTATAG |
| | CCTGAGCAGCACCCTGACCCTGAGCAAAGCGGATTATGAAAAACAT AAAGTGTATGCGTGCGAAGTGACCCATCAGGGCCTGAGCAGCCCG GTGACCAAAAGCTTTAACCGCGGCGAATGCCACCACCACCACCACC ACCAGGTGCAGCTGGTGGAAAGCGGCGGCGGCGTGGTGCAGCCG GGCCGCAGCCTGCGCCTGGATTGCAAAGCGAGCGGCATTACCTTT AGCAACAGCGGCATGCATTGGGTGCGCCAGGCGCCGGGCAAAGG CCTGGAATGGGTGGCGGTGATTTGGTATGATGGCAGCAAACGCTAT TATGCGGATAGCGTGAAAGGCCGCTTTACCATTAGCCGCGATAACA GCAAAAACACCCTGTTTCTGCAGATGAACAGCCTGCGCGCGGAAGA TACCGCGGTGTATTATTGCGCGACCAACGATGATTATTGGGGCCAG GGCACCCTGGTGACCGTGAGCAGCGCGAGCACCAAAGGCCCGAG CGTGTTTCCGCTGGCGCCGTGCAGCCGCAGCACCAGCGAAAGCAC CGCGGCGCTGGGCTGCCTGGTGAAAGATTATTTTCCGGAACCGGT GACCGTGAGCTGGAACAGCGGCGCGCTGACCAGCGGCGTGCATAC CTTTCCGGCGGTGCTGCAGAGCAGCGGCCTGTATAGCCTGAGCAG CGTGGTGACCGTGCCGAGCAGCAGCCTGGGCACCAAAACCTATAC CTGCAACGTGGATCATAAACCGAGCAACACCAAAGTGGATAAACGC GTGGAAAGCAAATATGGCCCGCCGTGCCCGCCGTGCCCGGCGCCG GAATTTCTGGGCGGCCCGAGCGTGTTTCTGTTTCCGCCGAAACCGA AAGATACCCTGATGATTAGCCGCACCCCGGAAGTGACCTGCGTGGT GGTGGATGTGAGCCAGGAAGATCCGGAAGTGCAGTTTAACTGGTAT GTGGATGGCGTGGAAGTGCATAACGCGAAAACCAAACCGCGCGAA GAACAGTTTAACAGCACCTATCGCGTGGTGAGCGTGCTGACCGTGC TGCATCAGGATTGGCTGAACGGCAAAGAATATAAATGCAAAGTGAG CAACAAAGGCCTGCCGAGCAGCATTGAAAAAACCATTAGCAAAGCG AAAGGCCAGCCGCGCGAACCGCAGGTGTATACCCTGCCGCCGAGC CAGGAAGAAATGACCAAAAACCAGGTGAGCCTGACCTGCCTGGTGA AAGGCTTTTATCCGAGCGATATTGCGGTGGAATGGGAAAGCAACGG CCAGCCGGAAAACAACTATAAAACCACCCCGCCGGTGCTGGATAGC GATGGCAGCTTTTTTCTGTATAGCCGCCTGACCGTGGATAAAAGCC GCTGGCAGGAAGGCAACGTGTTTAGCTGCAGCGTGATGCATGAAG CGCTGCATAACCATTATACCCAGAAAAGCCTGAGCCTGAGCCTGGG CAAATACCCATACGATGTTCCAGATTACGCTTATCCATATGATGTTC CAGATTATGCTTAA |
| SEQ ID NO:9 Nucleotide sequence of the Plasmid (minus **Nivolumab) with** CaMV35S Promoter, ***NOS pA** terminator,* RB T-DNA Repeat, CaMV35S Promoter, ***CaMV35s pA,*** Hygro, *LB T*- *DNA* Repeat | GCCAACATGGTGGAGCACGACACTCTCGTCTACTCCAAGAATATCA AAGATACAGTCTCAGAAGACCAAAGGGCTATTGAGACTTTTCAACAA AGGGTAATATCGGGAAACCTCCTCGGATTCCATTGCCCAGCTATCT GTCACTTCATCAAAAGGACAGTAGAAAAGGAAGGTGGCACCTACAA ATGCCATCATTGCGATAAAGGAAAGGCTATCGTTCAAGATGCCTCTG CCGACAGTGGTCCCAAAGATGGACCCCCACCCACGAGGAGCATCG TGGAAAAAGAAGACGTTCCAACCACGTCTTCAAAGCAAGTGGATTG ATGTGAACATGGTGGAGCACGACACTCTCGTCTACTCCAAGAATAT CAAAGATACAGTCTCAGAAGACCAAAGGGCTATTGAGACTTTTCAAC AAAGGGTAATATCGGGAAACCTCCTCGGATTCCATTGCCCAGCTAT CTGTCACTTCATCAAAAGGACAGTAGAAAAGGAAGGTGGCACCTAC AAATGCCATCATTGCGATAAAGGAAAGGCTATCGTTCAAGATGCCTC TGCCGACAGTGGTCCCAAAGATGGACCCCCACCCACGAGGAGCAT CGTGGAAAAAGAAGACGTTCCAACCACGTCTTCAAAGCAAGTGGAT TGATGTGATATCTCCACTGACGTAAGGGATGACGCACAATCCCACT ATCCTTCGCAAGACCCTTCCTCTATATAAGGAAGTTCATTTCATTTG GAGAGGACACGCTGA[NivolumabSequence]ACCCAGCTTTCTTGTA CAAAGTGGTAGCTCGAATTTCCCCGATCGTTCAAACATTTGGCAAT AAAGTTTCTTAAGATTGAATCCTGTTGCCGGTCTTGCGATGATTAT CATATAATTTCTGTTGAATTACGTTAAGCATGTAATAATTAACATGT AATGCATGACGTTATTTATGAGATGGGTTTTTATGATTAGAGTCCC |
| | GCAATTATACATTTAATACGCGATAGAAAACAAAATATAGCGCGC AAACTAGGATAAATTATCGCGCGCGGTGTCATCTATGTTACTAGAT CGGGAAAGCTTTTAAACTATCAGTGTTTGACAGGATATATTGGCGG GTAAACCTAAGAGAAAAGAGCGTTTATTAGAATAATCGGATATTTAA AAGGGCGTGAAAAGGTTTATCCGTTCGTCCATTTGTATGTGCATGCC AACCACAGGGTTCCCCTCGGGATCAAAGTACTTTGATCCAACCCCT CCGCTGCTATAGTGCAGTCGGCTTCTGACGTTCAGTGCAGCCGTCT TCTGAAAACGACATGTCGCACAAGTCCTAAGTTACGCGACAGGCTG CCGCCCTGCCCTTTTCCTGGCGTTTTCTTGTCGCGTGTTTTAGTCGC ATAAAGTAGAATACTTGCGACTAGAACCGGAGACATTACGCCATGAA CAAGAGCGCCGCCGCTGGCCTGCTGGGCTATGCCCGCGTCAGCAC CGACGACCAGGACTTGACCAACCAACGGGCCGAACTGCACGCGGC CGGCTGCACCAAGCTGTTTTCCGAGAAGATCACCGGCACCAGGCG CGACCGCCCGGAGCTGGCCAGGATGCTTGACCACCTACGCCCTGG CGACGTTGTGACAGTGACCAGGCTAGACCGCCTGGCCCGCAGCAC CCGCGACCTACTGGACATTGCCGAGCGCATCCAGGAGGCCGGCGC GGGCCTGCGTAGCCTGGCAGAGCCGTGGGCCGACACCACCACGC CGGCCGGCCGCATGGTGTTGACCGTGTTCGCCGGCATTGCCGAGT TCGAGCGTTCCCTAATCATCGACCGCACCCGGAGCGGGCGCGAGG CCGCCAAGGCCCGAGGCGTGAAGTTTGGCCCCCGCCCTACCCTCA CCCCGGCACAGATCGCGCACGCCCGCGAGCTGATCGACCAGGAAG GCCGCACCGTGAAAGAGGCGGCTGCACTGCTTGGCGTGCATCGCT CGACCCTGTACCGCGCACTTGAGCGCAGCGAGGAAGTGACGCCCA CCGAGGCCAGGCGGCGCGGTGCCTTCCGTGAGGACGCATTGACC GAGGCCGACGCCCTGGCGGCCGCCGAGAATGAACGCCAAGAGGA ACAAGCATGAAACCGCACCAGGACGGCCAGGACGAACCGTTTTTCA TTACCGAAGAGATCGAGGCGGAGATGATCGCGGCCGGGTACGTGT TCGAGCCGCCCGCGCACGTCTCAACCGTGCGGCTGCATGAAATCC TGGCCGGTTTGTCTGATGCCAAGCTGGCGGCCTGGCCGGCCAGCT TGGCCGCTGAAGAAACCGAGCGCCGCCGTCTAAAAAGGTGATGTG TATTTGAGTAAAACAGCTTGCGTCATGCGGTCGCTGCGTATATGATG CGATGAGTAAATAAACAAATACGCAAGGGGAACGCATGAAGGTTAT CGCTGTACTTAACCAGAAAGGCGGGTCAGGCAAGACGACCATCGC AACCCATCTAGCCCGCGCCCTGCAACTCGCCGGGGCCGATGTTCT GTTAGTCGATTCCGATCCCCAGGGCAGTGCCCGCGATTGGGCGGC CGTGCGGGAAGATCAACCGCTAACCGTTGTCGGCATCGACCGCCC GACGATTGACCGCGACGTGAAGGCCATCGGCCGGCGCGACTTCGT AGTGATCGACGGAGCGCCCCAGGCGGCGGACTTGGCTGTGTCCGC GATCAAGGCAGCCGACTTCGTGCTGATTCCGGTGCAGCCAAGCCCT TACGACATATGGGCCACCGCCGACCTGGTGGAGCTGGTTAAGCAG CGCATTGAGGTCACGGATGGAAGGCTACAAGCGGCCTTTGTCGTGT CGCGGGCGATCAAAGGCACGCGCATCGGCGGTGAGGTTGCCGAG GCGCTGGCCGGGTACGAGCTGCCCATTCTTGAGTCCCGTATCACG CAGCGCGTGAGCTACCCAGGCACTGCCGCCGCCGGCACAACCGTT CTTGAATCAGAACCCGAGGGCGACGCTGCCCGCGAGGTCCAGGCG CTGGCCGCTGAAATTAAATCAAAACTCATTTGAGTTAATGAGGTAAA GAGAAAATGAGCAAAAGCACAAACACGCTAAGTGCCGGCCGTCCGA GCGCACGCAGCAGCAAGGCTGCAACGTTGGCCAGCCTGGCAGACA CGCCAGCCATGAAGCGGGTCAACTTTCAGTTGCCGGCGGAGGATC ACACCAAGCTGAAGATGTACGCGGTACGCCAAGGCAAGACCATTAC CGAGCTGCTATCTGAATACATCGCGCAGCTACCAGAGTAAATGAGC AAATGAATAAATGAGTAGATGAATTTTAGCGGCTAAAGGAGGCGGC ATGGAAAATCAAGAACAACCAGGCACCGACGCCGTGGAATGCCCCA TGTGTGGAGGAACGGGCGGTTGGCCAGGCGTAAGCGGCTGGGTTG CCTGCCGGCCCTGCAATGGCACTGGAACCCCCAAGCCCGAGGAAT |
| | CGGCGTGAGCGGTCGCAAACCATCCGGCCCGGTACAAATCGGCGC GGCGCTGGGTGATGACCTGGTGGAGAAGTTGAAGGCCGCGCAGGC CGCCCAGCGGCAACGCATCGAGGCAGAAGCACGCCCCGGTGAATC GTGGCAAGCGGCCGCTGATCGAATCCGCAAAGAATCCCGGCAACC GCCGGCAGCCGGTGCGCCGTCGATTAGGAAGCCGCCCAAGGGCG ACGAGCAACCAGATTTTTTCGTTCCGATGCTCTATGACGTGGGCAC CCGCGATAGTCGCAGCATCATGGACGTGGCCGTTTTCCGTCTGTCG AAGCGTGACCGACGAGCTGGCGAGGTGATCCGCTACGAGCTTCCA GACGGGCACGTAGAGGTTTCCGCAGGGCCGGCCGGCATGGCCAG TGTGTGGGATTACGACCTGGTACTGATGGCGGTTTCCCATCTAACC GAATCCATGAACCGATACCGGGAAGGGAAGGGAGACAAGCCCGGC CGCGTGTTCCGTCCACACGTTGCGGACGTACTCAAGTTCTGCCGGC GAGCCGATGGCGGAAAGCAGAAAGACGACCTGGTAGAAACCTGCA TTCGGTTAAACACCACGCACGTTGCCATGCAGCGTACGAAGAAGGC CAAGAACGGCCGCCTGGTGACGGTATCCGAGGGTGAAGCCTTGAT TAGCCGCTACAAGATCGTAAAGAGCGAAACCGGGCGGCCGGAGTA CATCGAGATCGAGCTAGCTGATTGGATGTACCGCGAGATCACAGAA GGCAAGAACCCGGACGTGCTGACGGTTCACCCCGATTACTTTTTGA TCGATCCCGGCATCGGCCGTTTTCTCTACCGCCTGGCACGCCGCG CCGCAGGCAAGGCAGAAGCCAGATGGTTGTTCAAGACGATCTACGA ACGCAGTGGCAGCGCCGGAGAGTTCAAGAAGTTCTGTTTCACCGTG CGCAAGCTGATCGGGTCAAATGACCTGCCGGAGTACGATTTGAAGG AGGAGGCGGGGCAGGCTGGCCCGATCCTAGTCATGCGCTACCGCA ACCTGATCGAGGGCGAAGCATCCGCCGGTTCCTAATGTACGGAGC AGATGCTAGGGCAAATTGCCCTAGCAGGGGAAAAAGGTCGAAAAG GTCTCTTTCCTGTGGATAGCACGTACATTGGGAACCCAAAGCCGTA CATTGGGAACCGGAACCCGTACATTGGGAACCCAAAGCCGTACATT GGGAACCGGTCACACATGTAAGTGACTGATATAAAAGAGAAAAAAG GCGATTTTTCCGCCTAAAACTCTTTAAAACTTATTAAAACTCTTAAAA CCCGCCTGGCCTGTGCATAACTGTCTGGCCAGCGCACAGCCGAAG AGCTGCAAAAAGCGCCTACCCTTCGGTCGCTGCGCTCCCTACGCCC CGCCGCTTCGCGTCGGCCTATCGCGGCCGCTGGCCGCTCAAAAAT GGCTGGCCTACGGCCAGGCAATCTACCAGGGCGCGGACAAGCCGC GCCGTCGCCACTCGACCGCCGGCGCCCACATCAAGGCACCCTGCC TCGCGCGTTTCGGTGATGACGGTGAAAACCTCTGACACATGCAGCT CCCGGAGACGGTCACAGCTTGTCTGTAAGCGGATGCCGGGAGCAG ACAAGCCCGTCAGGGCGCGTCAGCGGGTGTTGGCGGGTGTCGGG GCGCAGCCATGACCCAGTCACGTAGCGATAGCGGAGTGTATACTG GCTTAACTATGCGGCATCAGAGCAGATTGTACTGAGAGTGCACCAT ATGCGGTGTGAAATACCGCACAGATGCGTAAGGAGAAAATACCGCA TCAGGCGCTCTTCCGCTTCCTCGCTCACTGACTCGCTGCGCTCGGT CGTTCGGCTGCGGCGAGCGGTATCAGCTCACTCAAAGGCGGTAAT ACGGTTATCCACAGAATCAGGGGATAACGCAGGAAAGAACATGTGA GCAAAAGGCCAGCAAAAGGCCAGGAACCGTAAAAAGGCCGCGTTG CTGGCGTTTTTCCATAGGCTCCGCCCCCCTGACGAGCATCACAAAA ATCGACGCTCAAGTCAGAGGTGGCGAAACCCGACAGGACTATAAAG ATACCAGGCGTTTCCCCCTGGAAGCTCCCTCGTGCGCTCTCCTGTT CCGACCCTGCCGCTTACCGGATACCTGTCCGCCTTTCTCCCTTCGG GAAGCGTGGCGCTTTCTCATAGCTCACGCTGTAGGTATCTCAGTTC GGTGTAGGTCGTTCGCTCCAAGCTGGGCTGTGTGCACGAACCCCC CGTTCAGCCCGACCGCTGCGCCTTATCCGGTAACTATCGTCTTGAG TCCAACCCGGTAAGACACGACTTATCGCCACTGGCAGCAGCCACTG GTAACAGGATTAGCAGAGCGAGGTATGTAGGCGGTGCTACAGAGTT CTTGAAGTGGTGGCCTAACTACGGCTACACTAGAAGGACAGTATTT GGTATCTGCGCTCTGCTGAAGCCAGTTACCTTCGGAAAAAGAGTTG |
| | GTAGCTCTTGATCCGGCAAACAAACCACCGCTGGTAGCGGTGGTTT TTTTGTTTGCAAGCAGCAGATTACGCGCAGAAAAAAAGGATCTCAAG AAGATCCTTTGATCTTTTCTACGGGGTCTGACGCTCAGTGGAACGAA AACTCACGTTAAGGGATTTTGGTCATGCATTCTAGGTACTAAAACAA TTCATCCAGTAAAATATAATATTTTATTTTCTCCCAATCAGGCTTGAT CCCCAGTAAGTCAAAAAATAGCTCGACATACTGTTCTTCCCCGATAT CCTCCCTGATCGACCGGACGCAGAAGGCAATGTCATACCACTTGTC CGCCCTGCCGCTTCTCCCAAGATCAATAAAGCCACTTACTTTGCCAT CTTTCACAAAGATGTTGCTGTCTCCCAGGTCGCCGTGGGAAAAGAC AAGTTCCTCTTCGGGCTTTTCCGTCTTTAAAAAATCATACAGCTCGC GCGGATCTTTAAATGGAGTGTCTTCTTCCCAGTTTTCGCAATCCACA TCGGCCAGATCGTTATTCAGTAAGTAATCCAATTCGGCTAAGCGGCT GTCTAAGCTATTCGTATAGGGACAATCCGATATGTCGATGGAGTGAA AGAGCCTGATGCACTCCGCATACAGCTCGATAATCTTTTCAGGGCTT TGTTCATCTTCATACTCTTCCGAGCAAAGGACGCCATCGGCCTCACT CATGAGCAGATTGCTCCAGCCATCATGCCGTTCAAAGTGCAGGACC TTTGGAACAGGCAGCTTTCCTTCCAGCCATAGCATCATGTCCTTTTC CCGTTCCACATCATAGGTGGTCCCTTTATACCGGCTGTCCGTCATTT TTAAATATAGGTTTTCATTTTCTCCCACCAGCTTATATACCTTAGCAG GAGACATTCCTTCCGTATCTTTTACGCAGCGGTATTTTTCGATCAGT TTTTTCAATTCCGGTGATATTCTCATTTTAGCCATTTATTATTTCCTTC CTCTTTTCTACAGTATTTAAAGATACCCCAAGAAGCTAATTATAACAA GACGAACTCCAATTCACTGTTCCTTGCATTCTAAAACCTTAAATACCA GAAAACAGCTTTTTCAAAGTTGTTTTCAAAGTTGGCGTATAACATAGT ATCGACGGAGCCGATTTTGAAACCGCGGTGATCACAGGCAGCAAC GCTCTGTCATCGTTACAATCAACATGCTACCCTCCGCGAGATCATCC GTGTTTCAAACCCGGCAGCTTAGTTGCCGTTCTTCCGAATAGCATC GGTAACATGAGCAAAGTCTGCCGCCTTACAACGGCTCTCCCGCTGA CGCCGTCCCGGACTGATGGGCTGCCTGTATCGAGTGGTGATTTTGT GCCGAGCTGCCGGTCGGGGAGCTGTTGGCTGGCTGGTGGCAGGA TATATTGTGGTGTAAACAAATTGACGCTTAGACAACTTAATAACACAT TGCGGACGTTTTTAATGTACTGAATTAACGCCGAATTGAATTCGAGC TCGGTACCGGGGGATCTGGATTTTAGTACTGGATTTTGGTTTTAGG AATTAGAAATTTTATTGATAGAAGTATTTTACAAATACAAATACATA CTAAGGGTTTCTTATATGCTCAACACATGAGCGAAACCCTATAGGA ACCCTAATTCCCTTATCTGGGAACTACTCACACATTATTATGGAGA AACTCGAGCTTGTCGATCGACAGATCCCGGTCGGCATCTACTCTAT TTCTTTGCCCTCGGACGAGTGCTGGGGCGTCGGTTTCCACTATCGG CGAGTACTTCTACACAGCCATCGGTCCAGACGGCCGCGCTTCTGCG GGCGATTTGTGTACGCCCGACAGTCCCGGCTCCGGATCGGACGAT TGCGTCGCATCGACCCTGCGCCCAAGCTGCATCATCGAAATTGCCG TCAACCAAGCTCTGATAGAGTTGGTCAAGACCAATGCGGAGCATAT ACGCCCGGAGTCGTGGCGATCCTGCAAGCTCCGGATGCCTCCGCT CGAAGTAGCGCGTCTGCTGCTCCATACAAGCCAACCACGGCCTCCA GAAGAAGATGTTGGCGACCTCGTATTGGGAATCCCCGAACATCGCC TCGCTCCAGTCAATGACCGCTGTTATGCGGCCATTGTCCGTCAGGA CATTGTTGGAGCCGAAATCCGCGTGCACGAGGTGCCGGACTTCGG GGCAGTCCTCGGCCCAAAGCATCAGCTCATCGAGAGCCTGCGCGA CGGACGCACTGACGGTGTCGTCCATCACAGTTTGCCAGTGATACAC ATGGGGATCAGCAATCGCGCATATGAAATCACGCCATGTAGTGTAT TGACCGATTCCTTGCGGTCCGAATGGGCCGAACCCGCTCGTCTGG CTAAGATCGGCCGCAGCGATCGCATCCATAGCCTCCGCGACCGGT TGTAGAACAGCGGGCAGTTCGGTTTCAGGCAGGTCTTGCAACGTGA CACCCTGTGAACGGCGGGAGATGCAATAGGTCAGGCTCTCGCTAAA CTCCCCAATGTCAAGCACTTCCGGAATCGGGAGCGCGGCCGATGC |
| | AAAGTGCCGATAAACATAACGATCTTTGTAGAAACCATCGGCGCAG CTATTTACCCGCAGGACATATCCACGCCCTCCTACATCGAAGCTGA AAGCACGAGATTCTTCGCCCTCCGAGAGCTGCATCAGGTCGGAGAC GCTGTCGAACTTTTCGATCAGAAACTTCTCGACAGACGTCGCGGTG AGTTCAGGCTTTTTCATATCTCATTGCCCCCCGGATCTGCGAAAGCT CGAGAGAGATAGATTTGTAGAGAGAGACTGGTGATTTCAGCGTGTC CTCTCCAAATGAAATGAACTTCCTTATATAGAGGAAGGGTCTTGCGA AGGATAGTGGGATTGTGCGTCATCCCTTACGTCAGTGGAGATATCA CATCAATCCACTTGCTTTGAAGACGTGGTTGGAACGTCTTCTTTTTC CACGATGCTCCTCGTGGGTGGGGGTCCATCTTTGGGACCACTGTC GGCAGAGGCATCTTGAACGATAGCCTTTCCTTTATCGCAATGATGG CATTTGTAGGTGCCACCTTCCTTTTCTACTGTCCTTTTGATGAAGTG ACAGATAGCTGGGCAATGGAATCCGAGGAGGTTTCCCGATATTACC CTTTGTTGAAAAGTCTCAATAGCCCTTTGGTCTTCTGAGACTGTATC TTTGATATTCTTGGAGTAGACGAGAGTGTCGTGCTCCACCATGTTCA CATCAATCCACTTGCTTTGAAGACGTGGTTGGAACGTCTTCTTTTTC CACGATGCTCCTCGTGGGTGGGGGTCCATCTTTGGGACCACTGTC GGCAGAGGCATCTTGAACGATAGCCTTTCCTTTATCGCAATGATGG CATTTGTAGGTGCCACCTTCCTTTTCTACTGTCCTTTTGATGAAGTG ACAGATAGCTGGGCAATGGAATCCGAGGAGGTTTCCCGATATTACC CTTTGTTGAAAAGTCTCAATAGCCCTTTGGTCTTCTGAGACTGTATC TTTGATATTCTTGGAGTAGACGAGAGTGTCGTGCTCCACCATGTTGG CGTACCCGGGGATCCTCTAGAGTCGACCTGCAGGCATGCAAGCT |
| SEQ ID NO: 10 Sequence of the Plasmids (minus Nivolumab) **with NOS** Promoter, ***NOS pA terminator,* RB T-DNA Repeat,** CaMV35S Promoter, **Hygro, *CaMV35s pA, LB T-DNA Repeat*** | GATCATGAGCGGAGAATTAAGGGAGTCACGTTATGACCCCCGCCGA TGACGCGGGACAAGCCGTTTTACGTTTGGAACTGACAGAACCGCAA CGTTGAAGGAGCCACTCAGCCGCGGGTTTCTGGAGTTTAATGAGCT AAGCACATACGTCAGAAACCATTATTGCGCGTTCAAAAGTCGCCTAA GGTCACTATCAGCTAGCAAATATTTCTTGTCAAAAATGCTCCACTGA CGTTCCATAAATTCCCCTCGGTATCCAATTAGAGTCTCATATTCACT CTCAATCCAAATAATCTGCA[NivolumabSequence]ACCCAGCTTTCT TGTACAAAGTGGTAGCTCGAATTTCCCCGATCGTTCAAACATTTGG CAATAAAGTTTCTTAAGATTGAATCCTGTTGCCGGTCTTGCGATGA TTATCATATAATTTCTGTTGAATTACGTTAAGCATGTAATAATTAAC ATGTAATGCATGACGTTATTTATGAGATGGGTTTTTATGATTAGAGT CCCGCAATTATACATTTAATACGCGATAGAAAACAAAATATAGCGC GCAAACTAGGATAAATTATCGCGCGCGGTGTCATCTATGTTACTAG ATCGGGAAAGCTTTTAAACTATCAGTGTTTGACAGGATATATTGGC GGGTAAACCTAAGAGAAAAGAGCGTTTATTAGAATAATCGGATATTT AAAAGGGCGTGAAAAGGTTTATCCGTTCGTCCATTTGTATGTGCATG CCAACCACAGGGTTCCCCTCGGGATCAAAGTACTTTGATCCAACCC CTCCGCTGCTATAGTGCAGTCGGCTTCTGACGTTCAGTGCAGCCGT CTTCTGAAAACGACATGTCGCACAAGTCCTAAGTTACGCGACAGGC TGCCGCCCTGCCCTTTTCCTGGCGTTTTCTTGTCGCGTGTTTTAGTC GCATAAAGTAGAATACTTGCGACTAGAACCGGAGACATTACGCCAT GAACAAGAGCGCCGCCGCTGGCCTGCTGGGCTATGCCCGCGTCAG CACCGACGACCAGGACTTGACCAACCAACGGGCCGAACTGCACGC GGCCGGCTGCACCAAGCTGTTTTCCGAGAAGATCACCGGCACCAG GCGCGACCGCCCGGAGCTGGCCAGGATGCTTGACCACCTACGCCC TGGCGACGTTGTGACAGTGACCAGGCTAGACCGCCTGGCCCGCAG CACCCGCGACCTACTGGACATTGCCGAGCGCATCCAGGAGGCCGG CGCGGGCCTGCGTAGCCTGGCAGAGCCGTGGGCCGACACCACCA CGCCGGCCGGCCGCATGGTGTTGACCGTGTTCGCCGGCATTGCCG AGTTCGAGCGTTCCCTAATCATCGACCGCACCCGGAGCGGGCGCG AGGCCGCCAAGGCCCGAGGCGTGAAGTTTGGCCCCCGCCCTACCC |
| | TCACCCCGGCACAGATCGCGCACGCCCGCGAGCTGATCGACCAGG AAGGCCGCACCGTGAAAGAGGCGGCTGCACTGCTTGGCGTGCATC GCTCGACCCTGTACCGCGCACTTGAGCGCAGCGAGGAAGTGACGC CCACCGAGGCCAGGCGGCGCGGTGCCTTCCGTGAGGACGCATTGA CCGAGGCCGACGCCCTGGCGGCCGCCGAGAATGAACGCCAAGAG GAACAAGCATGAAACCGCACCAGGACGGCCAGGACGAACCGTTTTT CATTACCGAAGAGATCGAGGCGGAGATGATCGCGGCCGGGTACGT GTTCGAGCCGCCCGCGCACGTCTCAACCGTGCGGCTGCATGAAAT CCTGGCCGGTTTGTCTGATGCCAAGCTGGCGGCCTGGCCGGCCAG CTTGGCCGCTGAAGAAACCGAGCGCCGCCGTCTAAAAAGGTGATGT GTATTTGAGTAAAACAGCTTGCGTCATGCGGTCGCTGCGTATATGAT GCGATGAGTAAATAAACAAATACGCAAGGGGAACGCATGAAGGTTA TCGCTGTACTTAACCAGAAAGGCGGGTCAGGCAAGACGACCATCGC AACCCATCTAGCCCGCGCCCTGCAACTCGCCGGGGCCGATGTTCT GTTAGTCGATTCCGATCCCCAGGGCAGTGCCCGCGATTGGGCGGC CGTGCGGGAAGATCAACCGCTAACCGTTGTCGGCATCGACCGCCC GACGATTGACCGCGACGTGAAGGCCATCGGCCGGCGCGACTTCGT AGTGATCGACGGAGCGCCCCAGGCGGCGGACTTGGCTGTGTCCGC GATCAAGGCAGCCGACTTCGTGCTGATTCCGGTGCAGCCAAGCCCT TACGACATATGGGCCACCGCCGACCTGGTGGAGCTGGTTAAGCAG CGCATTGAGGTCACGGATGGAAGGCTACAAGCGGCCTTTGTCGTGT CGCGGGCGATCAAAGGCACGCGCATCGGCGGTGAGGTTGCCGAG GCGCTGGCCGGGTACGAGCTGCCCATTCTTGAGTCCCGTATCACG CAGCGCGTGAGCTACCCAGGCACTGCCGCCGCCGGCACAACCGTT CTTGAATCAGAACCCGAGGGCGACGCTGCCCGCGAGGTCCAGGCG CTGGCCGCTGAAATTAAATCAAAACTCATTTGAGTTAATGAGGTAAA GAGAAAATGAGCAAAAGCACAAACACGCTAAGTGCCGGCCGTCCGA GCGCACGCAGCAGCAAGGCTGCAACGTTGGCCAGCCTGGCAGACA CGCCAGCCATGAAGCGGGTCAACTTTCAGTTGCCGGCGGAGGATC ACACCAAGCTGAAGATGTACGCGGTACGCCAAGGCAAGACCATTAC CGAGCTGCTATCTGAATACATCGCGCAGCTACCAGAGTAAATGAGC AAATGAATAAATGAGTAGATGAATTTTAGCGGCTAAAGGAGGCGGC ATGGAAAATCAAGAACAACCAGGCACCGACGCCGTGGAATGCCCCA TGTGTGGAGGAACGGGCGGTTGGCCAGGCGTAAGCGGCTGGGTTG CCTGCCGGCCCTGCAATGGCACTGGAACCCCCAAGCCCGAGGAAT CGGCGTGAGCGGTCGCAAACCATCCGGCCCGGTACAAATCGGCGC GGCGCTGGGTGATGACCTGGTGGAGAAGTTGAAGGCCGCGCAGGC CGCCCAGCGGCAACGCATCGAGGCAGAAGCACGCCCCGGTGAATC GTGGCAAGCGGCCGCTGATCGAATCCGCAAAGAATCCCGGCAACC GCCGGCAGCCGGTGCGCCGTCGATTAGGAAGCCGCCCAAGGGCG ACGAGCAACCAGATTTTTTCGTTCCGATGCTCTATGACGTGGGCAC CCGCGATAGTCGCAGCATCATGGACGTGGCCGTTTTCCGTCTGTCG AAGCGTGACCGACGAGCTGGCGAGGTGATCCGCTACGAGCTTCCA GACGGGCACGTAGAGGTTTCCGCAGGGCCGGCCGGCATGGCCAG TGTGTGGGATTACGACCTGGTACTGATGGCGGTTTCCCATCTAACC GAATCCATGAACCGATACCGGGAAGGGAAGGGAGACAAGCCCGGC CGCGTGTTCCGTCCACACGTTGCGGACGTACTCAAGTTCTGCCGGC GAGCCGATGGCGGAAAGCAGAAAGACGACCTGGTAGAAACCTGCA TTCGGTTAAACACCACGCACGTTGCCATGCAGCGTACGAAGAAGGC CAAGAACGGCCGCCTGGTGACGGTATCCGAGGGTGAAGCCTTGAT TAGCCGCTACAAGATCGTAAAGAGCGAAACCGGGCGGCCGGAGTA CATCGAGATCGAGCTAGCTGATTGGATGTACCGCGAGATCACAGAA GGCAAGAACCCGGACGTGCTGACGGTTCACCCCGATTACTTTTTGA TCGATCCCGGCATCGGCCGTTTTCTCTACCGCCTGGCACGCCGCG CCGCAGGCAAGGCAGAAGCCAGATGGTTGTTCAAGACGATCTACGA |
| | ACGCAGTGGCAGCGCCGGAGAGTTCAAGAAGTTCTGTTTCACCGTG CGCAAGCTGATCGGGTCAAATGACCTGCCGGAGTACGATTTGAAGG AGGAGGCGGGGCAGGCTGGCCCGATCCTAGTCATGCGCTACCGCA ACCTGATCGAGGGCGAAGCATCCGCCGGTTCCTAATGTACGGAGC AGATGCTAGGGCAAATTGCCCTAGCAGGGGAAAAAGGTCGAAAAG GTCTCTTTCCTGTGGATAGCACGTACATTGGGAACCCAAAGCCGTA CATTGGGAACCGGAACCCGTACATTGGGAACCCAAAGCCGTACATT GGGAACCGGTCACACATGTAAGTGACTGATATAAAAGAGAAAAAAG GCGATTTTTCCGCCTAAAACTCTTTAAAACTTATTAAAACTCTTAAAA CCCGCCTGGCCTGTGCATAACTGTCTGGCCAGCGCACAGCCGAAG AGCTGCAAAAAGCGCCTACCCTTCGGTCGCTGCGCTCCCTACGCCC CGCCGCTTCGCGTCGGCCTATCGCGGCCGCTGGCCGCTCAAAAAT GGCTGGCCTACGGCCAGGCAATCTACCAGGGCGCGGACAAGCCGC GCCGTCGCCACTCGACCGCCGGCGCCCACATCAAGGCACCCTGCC TCGCGCGTTTCGGTGATGACGGTGAAAACCTCTGACACATGCAGCT CCCGGAGACGGTCACAGCTTGTCTGTAAGCGGATGCCGGGAGCAG ACAAGCCCGTCAGGGCGCGTCAGCGGGTGTTGGCGGGTGTCGGG GCGCAGCCATGACCCAGTCACGTAGCGATAGCGGAGTGTATACTG GCTTAACTATGCGGCATCAGAGCAGATTGTACTGAGAGTGCACCAT ATGCGGTGTGAAATACCGCACAGATGCGTAAGGAGAAAATACCGCA TCAGGCGCTCTTCCGCTTCCTCGCTCACTGACTCGCTGCGCTCGGT CGTTCGGCTGCGGCGAGCGGTATCAGCTCACTCAAAGGCGGTAAT ACGGTTATCCACAGAATCAGGGGATAACGCAGGAAAGAACATGTGA GCAAAAGGCCAGCAAAAGGCCAGGAACCGTAAAAAGGCCGCGTTG CTGGCGTTTTTCCATAGGCTCCGCCCCCCTGACGAGCATCACAAAA ATCGACGCTCAAGTCAGAGGTGGCGAAACCCGACAGGACTATAAAG ATACCAGGCGTTTCCCCCTGGAAGCTCCCTCGTGCGCTCTCCTGTT CCGACCCTGCCGCTTACCGGATACCTGTCCGCCTTTCTCCCTTCGG GAAGCGTGGCGCTTTCTCATAGCTCACGCTGTAGGTATCTCAGTTC GGTGTAGGTCGTTCGCTCCAAGCTGGGCTGTGTGCACGAACCCCC CGTTCAGCCCGACCGCTGCGCCTTATCCGGTAACTATCGTCTTGAG TCCAACCCGGTAAGACACGACTTATCGCCACTGGCAGCAGCCACTG GTAACAGGATTAGCAGAGCGAGGTATGTAGGCGGTGCTACAGAGTT CTTGAAGTGGTGGCCTAACTACGGCTACACTAGAAGGACAGTATTT GGTATCTGCGCTCTGCTGAAGCCAGTTACCTTCGGAAAAAGAGTTG GTAGCTCTTGATCCGGCAAACAAACCACCGCTGGTAGCGGTGGTTT TTTTGTTTGCAAGCAGCAGATTACGCGCAGAAAAAAAGGATCTCAAG AAGATCCTTTGATCTTTTCTACGGGGTCTGACGCTCAGTGGAACGAA AACTCACGTTAAGGGATTTTGGTCATGCATTCTAGGTACTAAAACAA TTCATCCAGTAAAATATAATATTTTATTTTCTCCCAATCAGGCTTGAT CCCCAGTAAGTCAAAAAATAGCTCGACATACTGTTCTTCCCCGATAT CCTCCCTGATCGACCGGACGCAGAAGGCAATGTCATACCACTTGTC CGCCCTGCCGCTTCTCCCAAGATCAATAAAGCCACTTACTTTGCCAT CTTTCACAAAGATGTTGCTGTCTCCCAGGTCGCCGTGGGAAAAGAC AAGTTCCTCTTCGGGCTTTTCCGTCTTTAAAAAATCATACAGCTCGC GCGGATCTTTAAATGGAGTGTCTTCTTCCCAGTTTTCGCAATCCACA TCGGCCAGATCGTTATTCAGTAAGTAATCCAATTCGGCTAAGCGGCT GTCTAAGCTATTCGTATAGGGACAATCCGATATGTCGATGGAGTGAA AGAGCCTGATGCACTCCGCATACAGCTCGATAATCTTTTCAGGGCTT TGTTCATCTTCATACTCTTCCGAGCAAAGGACGCCATCGGCCTCACT CATGAGCAGATTGCTCCAGCCATCATGCCGTTCAAAGTGCAGGACC TTTGGAACAGGCAGCTTTCCTTCCAGCCATAGCATCATGTCCTTTTC CCGTTCCACATCATAGGTGGTCCCTTTATACCGGCTGTCCGTCATTT TTAAATATAGGTTTTCATTTTCTCCCACCAGCTTATATACCTTAGCAG GAGACATTCCTTCCGTATCTTTTACGCAGCGGTATTTTTCGATCAGT |
| | TTTTTCAATTCCGGTGATATTCTCATTTTAGCCATTTATTATTTCCTTC CTCTTTTCTACAGTATTTAAAGATACCCCAAGAAGCTAATTATAACAA GACGAACTCCAATTCACTGTTCCTTGCATTCTAAAACCTTAAATACCA GAAAACAGCTTTTTCAAAGTTGTTTTCAAAGTTGGCGTATAACATAGT ATCGACGGAGCCGATTTTGAAACCGCGGTGATCACAGGCAGCAAC GCTCTGTCATCGTTACAATCAACATGCTACCCTCCGCGAGATCATCC GTGTTTCAAACCCGGCAGCTTAGTTGCCGTTCTTCCGAATAGCATC GGTAACATGAGCAAAGTCTGCCGCCTTACAACGGCTCTCCCGCTGA CGCCGTCCCGGACTGATGGGCTGCCTGTATCGAGTGGTGATTTTGT GCCGAGCTGCCGGTCGGGGAGCTGTTGGCTGGCTGGTGGCAGGA TATATTGTGGTGTAAACAAATTGACGCTTAGACAACTTAATAACACAT TGCGGACGTTTTTAATGTACTGAATTAACGCCGAATTGAATTCGAGC TCGGTACCGGGGGATCTGGATTTTAGTACTGGATTTTGGTTTTAGG AATTAGAAATTTTATTGATAGAAGTATTTTACAAATACAAATACATA CTAAGGGTTTCTTATATGCTCAACACATGAGCGAAACCCTATAGGA ACCCTAATTCCCTTATCTGGGAACTACTCACACATTATTATGGAGA AACTCGAGCTTGTCGATCGACAGATCCCGGTCGGCATCTACTCTAT TTCTTTGCCCTCGGACGAGTGCTGGGGCGTCGGTTTCCACTATCGG CGAGTACTTCTACACAGCCATCGGTCCAGACGGCCGCGCTTCTGCG GGCGATTTGTGTACGCCCGACAGTCCCGGCTCCGGATCGGACGAT TGCGTCGCATCGACCCTGCGCCCAAGCTGCATCATCGAAATTGCCG TCAACCAAGCTCTGATAGAGTTGGTCAAGACCAATGCGGAGCATAT ACGCCCGGAGTCGTGGCGATCCTGCAAGCTCCGGATGCCTCCGCT CGAAGTAGCGCGTCTGCTGCTCCATACAAGCCAACCACGGCCTCCA GAAGAAGATGTTGGCGACCTCGTATTGGGAATCCCCGAACATCGCC TCGCTCCAGTCAATGACCGCTGTTATGCGGCCATTGTCCGTCAGGA CATTGTTGGAGCCGAAATCCGCGTGCACGAGGTGCCGGACTTCGG GGCAGTCCTCGGCCCAAAGCATCAGCTCATCGAGAGCCTGCGCGA CGGACGCACTGACGGTGTCGTCCATCACAGTTTGCCAGTGATACAC ATGGGGATCAGCAATCGCGCATATGAAATCACGCCATGTAGTGTAT TGACCGATTCCTTGCGGTCCGAATGGGCCGAACCCGCTCGTCTGG CTAAGATCGGCCGCAGCGATCGCATCCATAGCCTCCGCGACCGGT TGTAGAACAGCGGGCAGTTCGGTTTCAGGCAGGTCTTGCAACGTGA CACCCTGTGAACGGCGGGAGATGCAATAGGTCAGGCTCTCGCTAAA CTCCCCAATGTCAAGCACTTCCGGAATCGGGAGCGCGGCCGATGC AAAGTGCCGATAAACATAACGATCTTTGTAGAAACCATCGGCGCAG CTATTTACCCGCAGGACATATCCACGCCCTCCTACATCGAAGCTGA AAGCACGAGATTCTTCGCCCTCCGAGAGCTGCATCAGGTCGGAGAC GCTGTCGAACTTTTCGATCAGAAACTTCTCGACAGACGTCGCGGTG AGTTCAGGCTTTTTCATATCTCATTGCCCCCCGGATCTGCGAAAGCT CGAGAGAGATAGATTTGTAGAGAGAGACTGGTGATTTCAGCGTGTC CTCTCCAAATGAAATGAACTTCCTTATATAGAGGAAGGGTCTTGCGA AGGATAGTGGGATTGTGCGTCATCCCTTACGTCAGTGGAGATATCA CATCAATCCACTTGCTTTGAAGACGTGGTTGGAACGTCTTCTTTTTC CACGATGCTCCTCGTGGGTGGGGGTCCATCTTTGGGACCACTGTC GGCAGAGGCATCTTGAACGATAGCCTTTCCTTTATCGCAATGATGG CATTTGTAGGTGCCACCTTCCTTTTCTACTGTCCTTTTGATGAAGTG ACAGATAGCTGGGCAATGGAATCCGAGGAGGTTTCCCGATATTACC CTTTGTTGAAAAGTCTCAATAGCCCTTTGGTCTTCTGAGACTGTATC TTTGATATTCTTGGAGTAGACGAGAGTGTCGTGCTCCACCATGTTCA CATCAATCCACTTGCTTTGAAGACGTGGTTGGAACGTCTTCTTTTTC CACGATGCTCCTCGTGGGTGGGGGTCCATCTTTGGGACCACTGTC GGCAGAGGCATCTTGAACGATAGCCTTTCCTTTATCGCAATGATGG CATTTGTAGGTGCCACCTTCCTTTTCTACTGTCCTTTTGATGAAGTG ACAGATAGCTGGGCAATGGAATCCGAGGAGGTTTCCCGATATTACC |
| | CTTTGTTGAAAAGTCTCAATAGCCCTTTGGTCTTCTGAGACTGTATC TTTGATATTCTTGGAGTAGACGAGAGTGTCGTGCTCCACCATGTTGG CGTACCCGGGGATCCTCTAGAGTCGACCTGCAGGCATGCAAGCT |

## Claims

1. A plant material for use as medicament, wherein the plant material is derived from a plant of the *Brassica, Spinacia,* or *Lactuca* genus, genetically modified to express a biologically active recombinant protein and comprises the biologically active recombinant protein as an active ingredient.

2. The plant material for use according to claim 1, wherein the plant material is from an edible plant from the *Brassica* genus, preferably is from cabbage.

3. The plant material for use according to any of claims 1 or 2, wherein the plant material is a dried plant material, preferably a homogenate of dried plant material.

4. The plant material for use according to any of claims 1 to 3, wherein the biologically active recombinant protein is a multimeric protein.

5. The plant material for use according to any of claims 1 to 4, wherein the biologically active recombinant protein is an antibody or a fragment thereof.

6. The plant material for use according to any of claims 1 to 5, wherein the plant material is administered orally.

7. The plant material for use according to any one of claims 1 to 6, for use in a method for treatment of an intestinal disorder in a subject.

8. The plant material for use according to any one of claims 1 to 7, wherein the plant material delivers the biologically active recombinant protein, under active form, to the lumen or mucosal surface of the intestine of the subject.

9. The plant material for use according to any one of claim 1 to 8, wherein the intestinal disorder is selected from cancer, infectious diseases and auto-immune diseases.

10. The plant material for use according to any one of claim 1 to 9, wherein the intestinal disorder is colorectal cancer.

11. The plant material for use according to any one of claims 1 to 10, wherein the biologically active recombinant protein is an antibody selected from an anti-PD1 antibody, an anti-PDL1 antibody, an anti-CTLA4 antibody or a fragment thereof.

12. The plant material for use according to any one of claims 1 to 11, wherein the biologically active recombinant protein is selected from nivolumab and pembrolizumab.

13. A method for producing a plant material as defined in any of the preceding claims, the method comprising:
a) providing a plant of the *Brassica, Spinacia,* or *Lactuca* genus stably transformed to express the biologically active recombinant protein;
b) harvesting a plant material from the plant of step a).

14. The method of claim 13, wherein the method further comprises the steps of:
c) drying the plant material, preferably by freeze-drying or lyophilization, and/or
d) homogenizing the plant material.

15. A pharmaceutical composition comprising the plant material as defined in any of claims 1 to 12, and an orally acceptable support.
